# EUROPEAN PATENT APPLICATION

(11) **EP 3 299 460 A1**
(43) Date of publication of application: **28.03.2018**
(21) Application number: 16190577.3
(22) Date of filing: 26.09.2016
(51) Int. Cl.: C12N 15/09, A61K 45/00, C12N 9/00

(54) **NOVEL COMPOUNDS AND METHODS FOR MODULATING UBIQUITINATION**

(71) Applicant: Johann Wolfgang Goethe-Universität Frankfurt am Main, 60323 Frankfurt am Main (DE)
(72) Inventor: Dikic, Ivan, 60568 Frankfurt (DE); Bhogaraju, Sagar, 60437 Frankfurt (DE)
(74) Representative: Kuttenkeuler, David

(57) **Abstract**

The present invention pertains to the modification of the eukaryotic ubiquitin system by using the bacterial virulence factor SdeA and variants and mutants thereof. The invention is based on the phosphodiesterase activity of SdeA, which catalyzes a phospho-ribosylation (or simply "ribosylation") event of ubiquitin and ubiquitin-like proteins. The invention provides SdeA proteins and mutants without said phosphodiesterase activity, as well as medical applications of these recombinant proteins, their encoding nucleic acids, ribosylated ubiquitin proteins, and compounds which may act as selective or non-selective phosphodiesterase inhibitors.

SEQUENCE LISTING

<110>  Johann Wolfgang Goethe-Universitaet Frankfurt

<120>  NOVEL COMPOUNDS AND METHODS FOR MODULATING UBIQUITINATION

<130>  U30736EP

<160>  7

<170>  PatentIn version 3.5

<210>  1
<211>  1506
<212>  PRT
<213>  Legionella pneumophila

<400>  1

Met Ser Leu Gly Glu Ala Ile Met Pro Lys Tyr Val Glu Gly Val Glu
1               5                   10                  15

Leu Thr Gln Glu Gly Met His Ala Ile Phe Ala Arg Met Gly Tyr Gly
            20                  25                  30

Asp Ile Thr Ser Gly Ser Ile Tyr Asn Gly Val Pro Thr Ile Asp Thr
        35                  40                  45

Gly Ala Leu Asn Arg Gln Gly Phe Met Pro Val Leu Thr Gly Val Gly
    50                  55                  60

Pro His Arg Asp Ser Gly His Trp Ile Met Leu Ile Lys Gly Pro Gly
65                  70                  75                  80

Asn Gln Tyr Tyr Leu Phe Asp Pro Leu Gly Lys Thr Ser Gly Glu Gly
                85                  90                  95

Tyr Gln Asn Ile Leu Ala Ala Gln Leu Pro Met Gly Ser Thr Leu Ser
            100                 105                 110

Val Ile Pro Asn Gly Ser Gly Leu Asn Met Gly Leu Cys Gly Tyr Trp
        115                 120                 125

Val Ala Ser Ala Gly Leu Arg Ala His Gln Ala Leu Asn Gln His Asn
    130                 135                 140

Pro Pro Thr Leu Leu Asn Val Gly Gln Thr Ile Thr Asn Glu Met Arg
145                 150                 155                 160

Asn Glu Leu Asp His Asp Gly Tyr Arg Lys Ile Thr Gly Trp Leu Arg
                165                 170                 175

Ala Val Ala Asp Glu Phe Pro Glu Gly Asp Pro Gln Leu Asp Gly Lys
            180                 185                 190

Ala Leu Arg Glu Asn Thr Glu Lys Asp Leu Lys Ile Glu Ile Pro Thr
        195                 200                 205

Leu Val Leu Pro Gly Lys Asp Thr Ser Pro Lys Glu Met Ser Val Lys
    210                 215                 220

Pro Thr Ala Pro Gln Asp Lys Ser Val Pro Val Trp Asn Gly Phe Ser
225                 230                 235                 240

Leu Tyr Thr Asp Asp Thr Val Lys Ala Ala Ala Gln Tyr Ala Tyr Asp
                245                 250                 255

Asn Tyr Leu Gly Lys Pro Tyr Thr Gly Ser Val Glu Ser Ala Pro Ala
            260                 265                 270

Asn Phe Gly Gly Arg Met Val Tyr Arg Gln His His Gly Leu Ser His
        275                 280                 285

Thr Leu Arg Thr Met Ala Tyr Ala Glu Leu Ile Val Glu Glu Ala Arg
    290                 295                 300

Lys Ala Lys Leu Arg Gly Glu Thr Leu Gly Lys Phe Lys Asp Gly Arg
305                 310                 315                 320

Thr Ile Ala Asp Val Thr Pro Gln Glu Leu Lys Lys Ile Met Ile Ala
                325                 330                 335

Gln Ala Phe Phe Val Ala Gly Arg Asp Asp Glu Ala Ser Asp Ala Lys
            340                 345                 350

Asn Tyr Gln Lys Tyr His Glu Gln Ser Arg Asp Ala Phe Leu Lys Tyr
        355                 360                 365

Val Lys Asp Asn Glu Ser Thr Leu Ile Pro Asp Val Phe Lys Asp Gln
    370                 375                 380

Glu Asp Val Asn Phe Tyr Ala Arg Val Ile Glu Asp Lys Ser His Asp
385                 390                 395                 400

Trp Glu Ser Thr Pro Ala His Val Leu Ile Asn Gln Gly His Met Val
                405                 410                 415

Asp Leu Val Arg Val Lys Gln Pro Pro Glu Ser Phe Leu Gln Arg Tyr
            420                 425                 430

Phe Ser Ser Met Gln Arg Trp Ile Gly Ser Gln Ala Thr Glu Ala Val
        435                 440                 445

Phe Gly Ile Gln Arg Gln Phe Phe His Ala Thr Tyr Glu Val Val Ala
    450                 455                 460

Gly Phe Asp Ser Asp Asn Lys Glu Pro His Leu Val Val Ser Gly Leu
465                 470                 475                 480

Gly Arg Tyr Val Ile Gly Glu Asp Gly Gln Pro Ile Arg Glu Ala Pro
                485                 490                 495

Lys Lys Gly Gln Lys Glu Gly Asp Leu Lys Val Phe Pro Gln Thr Tyr
            500                 505                 510

Lys Leu Lys Glu Asn Glu Arg Leu Met Arg Val Asp Glu Phe Leu Lys
        515                 520                 525

Leu Pro Glu Ile Gln Asn Thr Phe Pro Gly Ser Gly Lys His Leu Gln
    530                 535                 540

Gly Gly Met Pro Gly Met Asn Glu Met Asp Tyr Trp Asn Arg Leu Asn
545                 550                 555                 560

Ser Leu Asn Arg Ala Arg Cys Glu Asn Asp Val Asp Phe Cys Leu Lys
                565                 570                 575

Gln Leu Gln Thr Ala His Asp Lys Ala Lys Ile Glu Pro Ile Lys Gln
            580                 585                 590

Ala Phe Gln Ser Ser Lys Gly Lys Glu Arg Arg Gln Pro Asn Val Asp
        595                 600                 605

Glu Ile Ala Ala Ala Arg Ile Ile Gln Gln Ile Leu Ala Asn Pro Asp
    610                 615                 620

Cys Ile His Asp Asp His Val Leu Ile Asn Gly Gln Lys Leu Glu Gln
625                 630                 635                 640

Gln Phe Phe Arg Asp Leu Leu Ala Lys Cys Glu Met Ala Val Val Gly
                645                 650                 655

Ser Leu Leu Asn Asp Thr Asp Ile Gly Asn Ile Asp Thr Leu Met Arg
            660                 665                 670

His Glu Lys Asp Thr Glu Phe His Ser Thr Asn Pro Glu Ala Val Pro
        675                 680                 685

Val Lys Ile Gly Glu Tyr Trp Ile Asn Asp Gln Arg Ile Asn Asn Ser
    690                 695                 700

Ser Gly Asn Ile Thr Gln Lys Lys His Asp Leu Ile Phe Leu Met Gln
705                 710                 715                 720

Asn Asp Ala Trp Tyr Phe Ser Arg Val Asn Ala Ile Ala Gln Asn Arg
                725                 730                 735

Asp Lys Gly Ser Thr Phe Lys Glu Val Leu Ile Thr Thr Leu Met Thr
            740                 745                 750

Pro Leu Thr Ser Lys Ala Leu Val Asp Thr Ser Gln Ala Lys Pro Pro
        755                 760                 765

Thr Arg Leu Phe Arg Gly Leu Asn Leu Ser Glu Glu Phe Thr Lys Gly
    770                 775                 780

Leu Ile Asp Gln Ala Asn Ala Met Ile Ala Asn Thr Thr Glu Arg Leu
785                 790                 795                 800

Phe Thr Asp His Ser Pro Glu Ala Phe Lys Gln Ile Lys Leu Asn Asp
                805                 810                 815

Leu Ser Lys Met Ser Gly Arg Thr Asn Ala Ser Thr Thr Thr Glu Ile
            820                 825                 830

Lys Leu Val Lys Glu Thr Trp Asp Ser Asn Val Ile Phe Glu Met Leu
        835                 840                 845

Asp Pro Asp Gly Leu Leu His Ser Lys Gln Val Gly Arg His Gly Glu
    850                 855                 860

Gly Thr Glu Ser Glu Phe Ser Val Tyr Leu Pro Glu Asp Val Ala Leu
865                 870                 875                 880

Val Pro Val Lys Val Thr Leu Asp Gly Lys Thr Gln Lys Gly Glu Asn
                885                 890                 895

Arg Tyr Val Phe Thr Phe Val Ala Val Lys Ser Pro Asp Phe Thr Pro
            900                 905                 910

Arg His Glu Ser Gly Tyr Ala Val Glu Pro Phe Leu Arg Met Gln Ala
        915                 920                 925

Ala Lys Leu Ala Glu Val Lys Ser Ser Ile Glu Lys Ala Gln Arg Ala

930                 935                 940

Pro Asp Leu Glu Thr Ile Phe Asn Leu Gln Asn Glu Val Glu Ala Val
945                 950                 955                 960

Gln Tyr Ser His Leu Ser Thr Gly Tyr Lys Asn Phe Leu Lys Asn Thr
                965                 970                 975

Val Gly Pro Val Leu Glu Asn Ser Leu Ser Gly Leu Met Glu Ser Asp
            980                 985                 990

Thr Asp Thr Leu Ser Lys Ala Leu  Ala Ala Phe Pro Ser  Asp Thr Gln
        995                 1000                1005

Trp Ser  Ala Phe Asn Phe Glu  Glu Ala Arg Gln Ala  Lys Arg Gln
    1010                1015                1020

Met Asp  Ala Ile Lys Gln Met  Val Gly Asn Lys Val  Val Leu Asp
    1025                1030                1035

Ala Leu  Thr Gln Cys Gln Asp  Ala Leu Glu Lys Gln  Asn Ile Ala
    1040                1045                1050

Gly Ala  Leu Asp Ala Leu Lys  Lys Ile Pro Ser Glu  Lys Glu Met
    1055                1060                1065

Gly Thr  Ile Arg Arg Glu Leu  Arg Glu Gln Ile Gln  Ser Ala Arg
    1070                1075                1080

Gln Glu  Leu Glu Ser Leu Gln  Arg Ala Val Val Thr  Pro Val Val
    1085                1090                1095

Thr Asp  Glu Lys Lys Val Arg  Glu Arg Tyr Asp Ala  Leu Ile Glu
    1100                1105                1110

Asn Thr  Ser Lys Lys Ile Thr  Glu Leu Glu Thr Gly  Lys Leu Pro
    1115                1120                1125

Asn Leu  Asp Ala Val Lys Lys  Gly Ile Ser Asn Leu  Ser Asn Leu
    1130                1135                1140

Lys Gln  Glu Val Thr Val Leu  Arg Asn Glu Lys Ile  Arg Met His
    1145                1150                1155

Val Gly  Thr Asp Lys Val Asp  Phe Ser Asp Val Glu  Lys Leu Glu
    1160                1165                1170

Gln Gln Ile Gln Val Ile Asp Thr Lys Leu Ala Asp Ala Tyr Leu
    1175                1180                1185

Leu Glu Val Thr Lys Gln Ile Ser Ala Leu Asp Asn Thr Lys Pro
    1190                1195                1200

Lys Asn Gln Thr Glu Leu Lys Thr Lys Ile Ala Ala Phe Leu Asp
    1205                1210                1215

Arg Thr Thr Asp Ile Glu Met Leu Arg Asn Glu Arg Ile Lys Lys
    1220                1225                1230

His Gly Ser Ser Lys Asp Pro Leu Asp Leu Ser Asp Leu Asp Lys
    1235                1240                1245

Leu Ser Gly Ser Leu Gln Arg Ile Asn Gln Ser Leu Val Ser Asp
    1250                1255                1260

Leu Ile Thr Thr Ile Arg Val Ser Ile Asn Gln Met Glu Ala Lys
    1265                1270                1275

Thr Phe His Glu Gln Glu Lys Glu Ile Gln Gln Asn Phe Glu Leu
    1280                1285                1290

Leu Ala Lys Leu Glu Lys Thr Leu Asp Lys Ser Lys Thr Ser Glu
    1295                1300                1305

Lys Leu Arg Glu Asp Ile Pro Lys Leu Asn Asp Leu Leu Val Ala
    1310                1315                1320

Lys Gln Lys Ala Tyr Pro Gln Met Val Gln Met Gln Leu Lys Ser
    1325                1330                1335

Glu Val Phe Val Thr Gln Leu Arg Glu Val Cys Gln Ala Asn His
    1340                1345                1350

Asp Asp Leu Asp Lys Thr Arg Asn Ala Arg Leu Arg Glu Leu Asp
    1355                1360                1365

Arg Leu Asp Arg Glu Ala Gly Ile Thr Arg Met Val Gly Asn Leu
    1370                1375                1380

Ile Trp Gly Leu Thr Asn Lys Val Gly Leu Thr Thr Asp Glu Arg
    1385                1390                1395

Leu Asp Ile Arg Thr Lys Gln Gln Ser Leu Ala Arg Phe Lys Asn
    1400                1405                1410

Glu Leu Phe Asn Asp Lys Ile Asp Thr Asp Gln Leu Ile Ser Asn
    1415                1420                1425

Leu Ala Arg Lys Arg Pro Ser Glu Leu Gln Glu Gly Leu Gly Ile
    1430                1435                1440

Ser Thr Asp Asn Ala Met Glu Leu His Leu Leu Leu Thr Glu Leu
    1445                1450                1455

Ala Gly Lys Thr Thr Ser Pro Asp Glu Leu Glu Glu Arg Met Lys
    1460                1465                1470

Ala Ile Asp Asp Ile Ser Thr Lys Ile Gly Arg Glu Pro Glu His
    1475                1480                1485

Leu Lys Phe Val Met Val Glu Glu Asp Glu Ser Asn Lys Lys Thr
    1490                1495                1500

Ile Gly Phe
    1505

<210> 2
<211> 1506
<212> PRT
<213> Legionella pneumophila

<400> 2

Met Ser Leu Gly Glu Ala Ile Met Pro Lys Tyr Val Glu Gly Val Glu
1               5                   10                  15

Leu Thr Gln Glu Gly Met His Ala Ile Phe Ala Arg Met Gly Tyr Gly
            20                  25                  30

Asp Ile Thr Ser Gly Ser Ile Tyr Asn Gly Val Pro Thr Ile Asp Thr
        35                  40                  45

Gly Ala Leu Asn Arg Gln Gly Phe Met Pro Val Leu Thr Gly Val Gly
    50                  55                  60

Pro His Arg Asp Ser Gly His Trp Ile Met Leu Ile Lys Gly Pro Gly
65                  70                  75                  80

Asn Gln Tyr Tyr Leu Phe Asp Pro Leu Gly Lys Thr Ser Gly Glu Gly
                85                  90                  95

Tyr Gln Asn Ile Leu Ala Ala Gln Leu Pro Met Gly Ser Thr Leu Ser
            100                 105                 110

Val Ile Pro Asn Gly Ser Gly Leu Asn Met Gly Leu Cys Gly Tyr Trp
        115                 120                 125

Val Ala Ser Ala Gly Leu Arg Ala His Gln Ala Leu Asn Gln His Asn
    130                 135                 140

Pro Pro Thr Leu Leu Asn Val Gly Gln Thr Ile Thr Asn Glu Met Arg
145                 150                 155                 160

Asn Glu Leu Asp His Asp Gly Tyr Arg Lys Ile Thr Gly Trp Leu Arg
                165                 170                 175

Ala Val Ala Asp Glu Phe Pro Glu Gly Asp Pro Gln Leu Asp Gly Lys
            180                 185                 190

Ala Leu Arg Glu Asn Thr Glu Lys Asp Leu Lys Ile Glu Ile Pro Thr
        195                 200                 205

Leu Val Leu Pro Gly Lys Asp Thr Ser Pro Lys Glu Met Ser Val Lys
    210                 215                 220

Pro Thr Ala Pro Gln Asp Lys Ser Val Pro Val Trp Asn Gly Phe Ser
225                 230                 235                 240

Leu Tyr Thr Asp Asp Thr Val Lys Ala Ala Ala Gln Tyr Ala Tyr Asp
                245                 250                 255

Asn Tyr Leu Gly Lys Pro Tyr Thr Gly Ser Val Glu Ser Ala Pro Ala
            260                 265                 270

Asn Phe Gly Gly Arg Met Val Tyr Arg Gln His His Gly Leu Ser His
        275                 280                 285

Thr Leu Arg Thr Met Ala Tyr Ala Glu Leu Ile Val Glu Glu Ala Arg
    290                 295                 300

Lys Ala Lys Leu Arg Gly Glu Thr Leu Gly Lys Phe Lys Asp Gly Arg
305                 310                 315                 320

Thr Ile Ala Asp Val Thr Pro Gln Glu Leu Lys Lys Ile Met Ile Ala
                325                 330                 335

Gln Ala Phe Phe Val Ala Gly Arg Asp Asp Glu Ala Ser Asp Ala Lys
            340                 345                 350

Asn Tyr Gln Lys Tyr His Glu Gln Ser Arg Asp Ala Phe Leu Lys Tyr
        355                 360                 365

Val Lys Asp Asn Glu Ser Thr Leu Ile Pro Asp Val Phe Lys Asp Gln
    370                 375                 380

Glu Asp Val Asn Phe Tyr Ala Arg Val Ile Glu Asp Lys Ser His Asp
385                 390                 395                 400

Trp Glu Ser Thr Pro Ala His Val Leu Ile Asn Gln Gly His Met Val
                405                 410                 415

Asp Leu Val Arg Val Lys Gln Pro Pro Glu Ser Phe Leu Gln Arg Tyr
            420                 425                 430

Phe Ser Ser Met Gln Arg Trp Ile Gly Ser Gln Ala Thr Glu Ala Val
        435                 440                 445

Phe Gly Ile Gln Arg Gln Phe Phe His Ala Thr Tyr Glu Val Val Ala
    450                 455                 460

Gly Phe Asp Ser Asp Asn Lys Glu Pro His Leu Val Val Ser Gly Leu
465                 470                 475                 480

Gly Arg Tyr Val Ile Gly Glu Asp Gly Gln Pro Ile Arg Glu Ala Pro
                485                 490                 495

Lys Lys Gly Gln Lys Glu Gly Asp Leu Lys Val Phe Pro Gln Thr Tyr
            500                 505                 510

Lys Leu Lys Glu Asn Glu Arg Leu Met Arg Val Asp Glu Phe Leu Lys
        515                 520                 525

Leu Pro Glu Ile Gln Asn Thr Phe Pro Gly Ser Gly Lys His Leu Gln
    530                 535                 540

Gly Gly Met Pro Gly Met Asn Glu Met Asp Tyr Trp Asn Arg Leu Asn
545                 550                 555                 560

Ser Leu Asn Arg Ala Arg Cys Glu Asn Asp Val Asp Phe Cys Leu Lys
                565                 570                 575

Gln Leu Gln Thr Ala His Asp Lys Ala Lys Ile Glu Pro Ile Lys Gln
            580                 585                 590

Ala Phe Gln Ser Ser Lys Gly Lys Glu Arg Arg Gln Pro Asn Val Asp
        595                 600                 605

Glu Ile Ala Ala Ala Arg Ile Ile Gln Gln Ile Leu Ala Asn Pro Asp

610                 615                 620

Cys Ile His Asp Asp His Val Leu Ile Asn Gly Gln Lys Leu Glu Gln
625                 630                 635                 640

Gln Phe Phe Arg Asp Leu Leu Ala Lys Cys Glu Met Ala Val Val Gly
                645                 650                 655

Ser Leu Leu Asn Asp Thr Asp Ile Gly Asn Ile Asp Thr Leu Met Arg
            660                 665                 670

His Glu Lys Asp Thr Glu Phe His Ser Thr Asn Pro Glu Ala Val Pro
        675                 680                 685

Val Lys Ile Gly Glu Tyr Trp Ile Asn Asp Gln Arg Ile Asn Asn Ser
    690                 695                 700

Ser Gly Asn Ile Thr Gln Lys Lys His Asp Leu Ile Phe Leu Met Gln
705                 710                 715                 720

Asn Asp Ala Trp Tyr Phe Ser Arg Val Asn Ala Ile Ala Gln Asn Arg
                725                 730                 735

Asp Lys Gly Ser Thr Phe Lys Glu Val Leu Ile Thr Thr Leu Met Thr
            740                 745                 750

Pro Leu Thr Ser Lys Ala Leu Val Asp Thr Ser Gln Ala Lys Pro Pro
        755                 760                 765

Thr Arg Leu Phe Arg Gly Leu Asn Leu Ser Glu Glu Phe Thr Lys Gly
    770                 775                 780

Leu Ile Asp Gln Ala Asn Ala Met Ile Ala Asn Thr Thr Glu Arg Leu
785                 790                 795                 800

Phe Thr Asp His Ser Pro Glu Ala Phe Lys Gln Ile Lys Leu Asn Asp
                805                 810                 815

Leu Ser Lys Met Ser Gly Arg Thr Asn Ala Ser Thr Thr Thr Glu Ile
            820                 825                 830

Lys Leu Val Lys Glu Thr Trp Asp Ser Asn Val Ile Phe Glu Met Leu
        835                 840                 845

Asp Pro Asp Gly Leu Leu His Ser Lys Gln Val Gly Arg His Gly Glu
    850                 855                 860

Gly Thr Ala Ser Ala Phe Ser Val Tyr Leu Pro Glu Asp Val Ala Leu
865                 870                 875                 880

Val Pro Val Lys Val Thr Leu Asp Gly Lys Thr Gln Lys Gly Glu Asn
                885                 890                 895

Arg Tyr Val Phe Thr Phe Val Ala Val Lys Ser Pro Asp Phe Thr Pro
            900                 905                 910

Arg His Glu Ser Gly Tyr Ala Val Glu Pro Phe Leu Arg Met Gln Ala
        915                 920                 925

Ala Lys Leu Ala Glu Val Lys Ser Ser Ile Glu Lys Ala Gln Arg Ala
    930                 935                 940

Pro Asp Leu Glu Thr Ile Phe Asn Leu Gln Asn Glu Val Glu Ala Val
945                 950                 955                 960

Gln Tyr Ser His Leu Ser Thr Gly Tyr Lys Asn Phe Leu Lys Asn Thr
                965                 970                 975

Val Gly Pro Val Leu Glu Asn Ser Leu Ser Gly Leu Met Glu Ser Asp
            980                 985                 990

Thr Asp Thr Leu Ser Lys Ala Leu  Ala Ala Phe Pro Ser  Asp Thr Gln
        995                 1000                1005

Trp Ser  Ala Phe Asn Phe Glu  Glu Ala Arg Gln Ala  Lys Arg Gln
    1010                1015                1020

Met Asp  Ala Ile Lys Gln Met  Val Gly Asn Lys Val  Val Leu Asp
    1025                1030                1035

Ala Leu  Thr Gln Cys Gln Asp  Ala Leu Glu Lys Gln  Asn Ile Ala
    1040                1045                1050

Gly Ala  Leu Asp Ala Leu Lys  Lys Ile Pro Ser Glu  Lys Glu Met
    1055                1060                1065

Gly Thr  Ile Arg Arg Glu Leu  Arg Glu Gln Ile Gln  Ser Ala Arg
    1070                1075                1080

Gln Glu  Leu Glu Ser Leu Gln  Arg Ala Val Val Thr  Pro Val Val
    1085                1090                1095

Thr Asp  Glu Lys Lys Val Arg  Glu Arg Tyr Asp Ala  Leu Ile Glu
    1100                1105                1110

Asn Thr Ser Lys Lys Ile Thr Glu Leu Glu Thr Gly Lys Leu Pro
    1115                1120                1125

Asn Leu Asp Ala Val Lys Lys Gly Ile Ser Asn Leu Ser Asn Leu
    1130                1135                1140

Lys Gln Glu Val Thr Val Leu Arg Asn Glu Lys Ile Arg Met His
    1145                1150                1155

Val Gly Thr Asp Lys Val Asp Phe Ser Asp Val Glu Lys Leu Glu
    1160                1165                1170

Gln Gln Ile Gln Val Ile Asp Thr Lys Leu Ala Asp Ala Tyr Leu
    1175                1180                1185

Leu Glu Val Thr Lys Gln Ile Ser Ala Leu Asp Asn Thr Lys Pro
    1190                1195                1200

Lys Asn Gln Thr Glu Leu Lys Thr Lys Ile Ala Ala Phe Leu Asp
    1205                1210                1215

Arg Thr Thr Asp Ile Glu Met Leu Arg Asn Glu Arg Ile Lys Lys
    1220                1225                1230

His Gly Ser Ser Lys Asp Pro Leu Asp Leu Ser Asp Leu Asp Lys
    1235                1240                1245

Leu Ser Gly Ser Leu Gln Arg Ile Asn Gln Ser Leu Val Ser Asp
    1250                1255                1260

Leu Ile Thr Thr Ile Arg Val Ser Ile Asn Gln Met Glu Ala Lys
    1265                1270                1275

Thr Phe His Glu Gln Glu Lys Glu Ile Gln Gln Asn Phe Glu Leu
    1280                1285                1290

Leu Ala Lys Leu Glu Lys Thr Leu Asp Lys Ser Lys Thr Ser Glu
    1295                1300                1305

Lys Leu Arg Glu Asp Ile Pro Lys Leu Asn Asp Leu Leu Val Ala
    1310                1315                1320

Lys Gln Lys Ala Tyr Pro Gln Met Val Gln Met Gln Leu Lys Ser
    1325                1330                1335

Glu Val Phe Val Thr Gln Leu Arg Glu Val Cys Gln Ala Asn His
    1340                1345                1350

Asp Asp Leu Asp Lys Thr Arg Asn Ala Arg Leu Arg Glu Leu Asp
    1355                1360                1365

Arg Leu Asp Arg Glu Ala Gly Ile Thr Arg Met Val Gly Asn Leu
    1370                1375                1380

Ile Trp Gly Leu Thr Asn Lys Val Gly Leu Thr Thr Asp Glu Arg
    1385                1390                1395

Leu Asp Ile Arg Thr Lys Gln Gln Ser Leu Ala Arg Phe Lys Asn
    1400                1405                1410

Glu Leu Phe Asn Asp Lys Ile Asp Thr Asp Gln Leu Ile Ser Asn
    1415                1420                1425

Leu Ala Arg Lys Arg Pro Ser Glu Leu Gln Glu Gly Leu Gly Ile
    1430                1435                1440

Ser Thr Asp Asn Ala Met Glu Leu His Leu Leu Leu Thr Glu Leu
    1445                1450                1455

Ala Gly Lys Thr Thr Ser Pro Asp Glu Leu Glu Glu Arg Met Lys
    1460                1465                1470

Ala Ile Asp Asp Ile Ser Thr Lys Ile Gly Arg Glu Pro Glu His
    1475                1480                1485

Leu Lys Phe Val Met Val Glu Glu Asp Glu Ser Asn Lys Lys Thr
    1490                1495                1500

Ile Gly Phe
    1505

<210> 3
<211> 806
<212> PRT
<213> Legionella pneumophila

<400> 3

Lys Asp Leu Lys Ile Glu Ile Pro Thr Leu Val Leu Pro Gly Lys Asp
1               5                   10                  15

Thr Ser Pro Lys Glu Met Ser Val Lys Pro Thr Ala Pro Gln Asp Lys
            20                  25                  30

Ser Val Pro Val Trp Asn Gly Phe Ser Leu Tyr Thr Asp Asp Thr Val
        35                  40                  45

Lys Ala Ala Ala Gln Tyr Ala Tyr Asp Asn Tyr Leu Gly Lys Pro Tyr
    50                  55                  60

Thr Gly Ser Val Glu Ser Ala Pro Ala Asn Phe Gly Gly Arg Met Val
65                  70                  75                  80

Tyr Arg Gln His His Gly Leu Ser His Thr Leu Arg Thr Met Ala Tyr
                85                  90                  95

Ala Glu Leu Ile Val Glu Glu Ala Arg Lys Ala Lys Leu Arg Gly Glu
            100                 105                 110

Thr Leu Gly Lys Phe Lys Asp Gly Arg Thr Ile Ala Asp Val Thr Pro
        115                 120                 125

Gln Glu Leu Lys Lys Ile Met Ile Ala Gln Ala Phe Phe Val Ala Gly
    130                 135                 140

Arg Asp Asp Glu Ala Ser Asp Ala Lys Asn Tyr Gln Lys Tyr His Glu
145                 150                 155                 160

Gln Ser Arg Asp Ala Phe Leu Lys Tyr Val Lys Asp Asn Glu Ser Thr
                165                 170                 175

Leu Ile Pro Asp Val Phe Lys Asp Gln Glu Asp Val Asn Phe Tyr Ala
            180                 185                 190

Arg Val Ile Glu Asp Lys Ser His Asp Trp Glu Ser Thr Pro Ala His
        195                 200                 205

Val Leu Ile Asn Gln Gly His Met Val Asp Leu Val Arg Val Lys Gln
    210                 215                 220

Pro Pro Glu Ser Phe Leu Gln Arg Tyr Phe Ser Ser Met Gln Arg Trp
225                 230                 235                 240

Ile Gly Ser Gln Ala Thr Glu Ala Val Phe Gly Ile Gln Arg Gln Phe
                245                 250                 255

Phe His Ala Thr Tyr Glu Val Val Ala Gly Phe Asp Ser Asp Asn Lys
            260                 265                 270

Glu Pro His Leu Val Val Ser Gly Leu Gly Arg Tyr Val Ile Gly Glu
        275                 280                 285

Asp Gly Gln Pro Ile Arg Glu Ala Pro Lys Lys Gly Gln Lys Glu Gly

290                 295                 300

Asp Leu Lys Val Phe Pro Gln Thr Tyr Lys Leu Lys Glu Asn Glu Arg
305                 310                 315                 320

Leu Met Arg Val Asp Glu Phe Leu Lys Leu Pro Glu Ile Gln Asn Thr
                325                 330                 335

Phe Pro Gly Ser Gly Lys His Leu Gln Gly Gly Met Pro Gly Met Asn
            340                 345                 350

Glu Met Asp Tyr Trp Asn Arg Leu Asn Ser Leu Asn Arg Ala Arg Cys
        355                 360                 365

Glu Asn Asp Val Asp Phe Cys Leu Lys Gln Leu Gln Thr Ala His Asp
    370                 375                 380

Lys Ala Lys Ile Glu Pro Ile Lys Gln Ala Phe Gln Ser Ser Lys Gly
385                 390                 395                 400

Lys Glu Arg Arg Gln Pro Asn Val Asp Glu Ile Ala Ala Ala Arg Ile
                405                 410                 415

Ile Gln Gln Ile Leu Ala Asn Pro Asp Cys Ile His Asp Asp His Val
            420                 425                 430

Leu Ile Asn Gly Gln Lys Leu Glu Gln Gln Phe Phe Arg Asp Leu Leu
        435                 440                 445

Ala Lys Cys Glu Met Ala Val Val Gly Ser Leu Leu Asn Asp Thr Asp
    450                 455                 460

Ile Gly Asn Ile Asp Thr Leu Met Arg His Glu Lys Asp Thr Glu Phe
465                 470                 475                 480

His Ser Thr Asn Pro Glu Ala Val Pro Val Lys Ile Gly Glu Tyr Trp
                485                 490                 495

Ile Asn Asp Gln Arg Ile Asn Asn Ser Ser Gly Asn Ile Thr Gln Lys
            500                 505                 510

Lys His Asp Leu Ile Phe Leu Met Gln Asn Asp Ala Trp Tyr Phe Ser
        515                 520                 525

Arg Val Asn Ala Ile Ala Gln Asn Arg Asp Lys Gly Ser Thr Phe Lys
    530                 535                 540

Glu Val Leu Ile Thr Thr Leu Met Thr Pro Leu Thr Ser Lys Ala Leu
545                 550                 555                 560

Val Asp Thr Ser Gln Ala Lys Pro Pro Thr Arg Leu Phe Arg Gly Leu
                565                 570                 575

Asn Leu Ser Glu Glu Phe Thr Lys Gly Leu Ile Asp Gln Ala Asn Ala
            580                 585                 590

Met Ile Ala Asn Thr Thr Glu Arg Leu Phe Thr Asp His Ser Pro Glu
        595                 600                 605

Ala Phe Lys Gln Ile Lys Leu Asn Asp Leu Ser Lys Met Ser Gly Arg
    610                 615                 620

Thr Asn Ala Ser Thr Thr Thr Glu Ile Lys Leu Val Lys Glu Thr Trp
625                 630                 635                 640

Asp Ser Asn Val Ile Phe Glu Met Leu Asp Pro Asp Gly Leu Leu His
                645                 650                 655

Ser Lys Gln Val Gly Arg His Gly Glu Gly Thr Glu Ser Glu Phe Ser
            660                 665                 670

Val Tyr Leu Pro Glu Asp Val Ala Leu Val Pro Val Lys Val Thr Leu
        675                 680                 685

Asp Gly Lys Thr Gln Lys Gly Glu Asn Arg Tyr Val Phe Thr Phe Val
    690                 695                 700

Ala Val Lys Ser Pro Asp Phe Thr Pro Arg His Glu Ser Gly Tyr Ala
705                 710                 715                 720

Val Glu Pro Phe Leu Arg Met Gln Ala Ala Lys Leu Ala Glu Val Lys
                725                 730                 735

Ser Ser Ile Glu Lys Ala Gln Arg Ala Pro Asp Leu Glu Thr Ile Phe
            740                 745                 750

Asn Leu Gln Asn Glu Val Glu Ala Val Gln Tyr Ser His Leu Ser Thr
        755                 760                 765

Gly Tyr Lys Asn Phe Leu Lys Asn Thr Val Gly Pro Val Leu Glu Asn
    770                 775                 780

Ser Leu Ser Gly Leu Met Glu Ser Asp Thr Asp Thr Leu Ser Lys Ala
785                 790                 795                 800

Leu Ala Ala Phe Pro Ser
                805

<210>  4
<211>  806
<212>  PRT
<213>  Legionella pneumophila

<400>  4

Lys Asp Leu Lys Ile Glu Ile Pro Thr Leu Val Leu Pro Gly Lys Asp
1               5                   10                  15

Thr Ser Pro Lys Glu Met Ser Val Lys Pro Thr Ala Pro Gln Asp Lys
            20                  25                  30

Ser Val Pro Val Trp Asn Gly Phe Ser Leu Tyr Thr Asp Asp Thr Val
        35                  40                  45

Lys Ala Ala Ala Gln Tyr Ala Tyr Asp Asn Tyr Leu Gly Lys Pro Tyr
    50                  55                  60

Thr Gly Ser Val Glu Ser Ala Pro Ala Asn Phe Gly Gly Arg Met Val
65                  70                  75                  80

Tyr Arg Gln His Ala Gly Leu Ser His Thr Leu Arg Thr Met Ala Tyr
                85                  90                  95

Ala Glu Leu Ile Val Glu Glu Ala Arg Lys Ala Lys Leu Arg Gly Glu
            100                 105                 110

Thr Leu Gly Lys Phe Lys Asp Gly Arg Thr Ile Ala Asp Val Thr Pro
        115                 120                 125

Gln Glu Leu Lys Lys Ile Met Ile Ala Gln Ala Phe Phe Val Ala Gly
    130                 135                 140

Arg Asp Asp Glu Ala Ser Asp Ala Lys Asn Tyr Gln Lys Tyr His Glu
145                 150                 155                 160

Gln Ser Arg Asp Ala Phe Leu Lys Tyr Val Lys Asp Asn Glu Ser Thr
                165                 170                 175

Leu Ile Pro Asp Val Phe Lys Asp Gln Glu Asp Val Asn Phe Tyr Ala
            180                 185                 190

Arg Val Ile Glu Asp Lys Ser His Asp Trp Glu Ser Thr Pro Ala His
        195                 200                 205

Val Leu Ile Asn Gln Gly His Met Val Asp Leu Val Arg Val Lys Gln
    210                 215                 220

Pro Pro Glu Ser Phe Leu Gln Arg Tyr Phe Ser Ser Met Gln Arg Trp
225                 230                 235                 240

Ile Gly Ser Gln Ala Thr Glu Ala Val Phe Gly Ile Gln Arg Gln Phe
                245                 250                 255

Phe His Ala Thr Tyr Glu Val Val Ala Gly Phe Asp Ser Asp Asn Lys
            260                 265                 270

Glu Pro His Leu Val Val Ser Gly Leu Gly Arg Tyr Val Ile Gly Glu
        275                 280                 285

Asp Gly Gln Pro Ile Arg Glu Ala Pro Lys Lys Gly Gln Lys Glu Gly
    290                 295                 300

Asp Leu Lys Val Phe Pro Gln Thr Tyr Lys Leu Lys Glu Asn Glu Arg
305                 310                 315                 320

Leu Met Arg Val Asp Glu Phe Leu Lys Leu Pro Glu Ile Gln Asn Thr
                325                 330                 335

Phe Pro Gly Ser Gly Lys His Leu Gln Gly Gly Met Pro Gly Met Asn
            340                 345                 350

Glu Met Asp Tyr Trp Asn Arg Leu Asn Ser Leu Asn Arg Ala Arg Cys
        355                 360                 365

Glu Asn Asp Val Asp Phe Cys Leu Lys Gln Leu Gln Thr Ala His Asp
    370                 375                 380

Lys Ala Lys Ile Glu Pro Ile Lys Gln Ala Phe Gln Ser Ser Lys Gly
385                 390                 395                 400

Lys Glu Arg Arg Gln Pro Asn Val Asp Glu Ile Ala Ala Ala Arg Ile
                405                 410                 415

Ile Gln Gln Ile Leu Ala Asn Pro Asp Cys Ile His Asp Asp His Val
            420                 425                 430

Leu Ile Asn Gly Gln Lys Leu Glu Gln Gln Phe Phe Arg Asp Leu Leu
        435                 440                 445

Ala Lys Cys Glu Met Ala Val Val Gly Ser Leu Leu Asn Asp Thr Asp
    450                 455                 460

Ile Gly Asn Ile Asp Thr Leu Met Arg His Glu Lys Asp Thr Glu Phe
465                 470                 475                 480

His Ser Thr Asn Pro Glu Ala Val Pro Val Lys Ile Gly Glu Tyr Trp
                485                 490                 495

Ile Asn Asp Gln Arg Ile Asn Asn Ser Ser Gly Asn Ile Thr Gln Lys
            500                 505                 510

Lys His Asp Leu Ile Phe Leu Met Gln Asn Asp Ala Trp Tyr Phe Ser
        515                 520                 525

Arg Val Asn Ala Ile Ala Gln Asn Arg Asp Lys Gly Ser Thr Phe Lys
    530                 535                 540

Glu Val Leu Ile Thr Thr Leu Met Thr Pro Leu Thr Ser Lys Ala Leu
545                 550                 555                 560

Val Asp Thr Ser Gln Ala Lys Pro Pro Thr Arg Leu Phe Arg Gly Leu
                565                 570                 575

Asn Leu Ser Glu Glu Phe Thr Lys Gly Leu Ile Asp Gln Ala Asn Ala
            580                 585                 590

Met Ile Ala Asn Thr Thr Glu Arg Leu Phe Thr Asp His Ser Pro Glu
        595                 600                 605

Ala Phe Lys Gln Ile Lys Leu Asn Asp Leu Ser Lys Met Ser Gly Arg
    610                 615                 620

Thr Asn Ala Ser Thr Thr Thr Glu Ile Lys Leu Val Lys Glu Thr Trp
625                 630                 635                 640

Asp Ser Asn Val Ile Phe Glu Met Leu Asp Pro Asp Gly Leu Leu His
                645                 650                 655

Ser Lys Gln Val Gly Arg His Gly Glu Gly Thr Glu Ser Glu Phe Ser
            660                 665                 670

Val Tyr Leu Pro Glu Asp Val Ala Leu Val Pro Val Lys Val Thr Leu
        675                 680                 685

Asp Gly Lys Thr Gln Lys Gly Glu Asn Arg Tyr Val Phe Thr Phe Val
    690                 695                 700

Ala Val Lys Ser Pro Asp Phe Thr Pro Arg His Glu Ser Gly Tyr Ala

705                 710                 715                 720

Val Glu Pro Phe Leu Arg Met Gln Ala Ala Lys Leu Ala Glu Val Lys
                725                 730                 735

Ser Ser Ile Glu Lys Ala Gln Arg Ala Pro Asp Leu Glu Thr Ile Phe
            740                 745                 750

Asn Leu Gln Asn Glu Val Glu Ala Val Gln Tyr Ser His Leu Ser Thr
        755                 760                 765

Gly Tyr Lys Asn Phe Leu Lys Asn Thr Val Gly Pro Val Leu Glu Asn
    770                 775                 780

Ser Leu Ser Gly Leu Met Glu Ser Asp Thr Asp Thr Leu Ser Lys Ala
785                 790                 795                 800

Leu Ala Ala Phe Pro Ser
                805

<210>  5
<211>  806
<212>  PRT
<213>  Legionella pneumophila

<400>  5

Lys Asp Leu Lys Ile Glu Ile Pro Thr Leu Val Leu Pro Gly Lys Asp
1               5                   10                  15

Thr Ser Pro Lys Glu Met Ser Val Lys Pro Thr Ala Pro Gln Asp Lys
            20                  25                  30

Ser Val Pro Val Trp Asn Gly Phe Ser Leu Tyr Thr Asp Asp Thr Val
        35                  40                  45

Lys Ala Ala Ala Gln Tyr Ala Tyr Asp Asn Tyr Leu Gly Lys Pro Tyr
    50                  55                  60

Thr Gly Ser Val Glu Ser Ala Pro Ala Asn Phe Gly Gly Arg Met Val
65                  70                  75                  80

Tyr Arg Gln His His Gly Leu Ser His Thr Leu Arg Thr Met Ala Tyr
                85                  90                  95

Ala Glu Leu Ile Val Glu Glu Ala Arg Lys Ala Lys Leu Arg Gly Glu
            100                 105                 110

Thr Leu Gly Lys Phe Lys Asp Gly Arg Thr Ile Ala Asp Val Thr Pro

115                 120                 125

Gln Glu Leu Lys Lys Ile Met Ile Ala Gln Ala Phe Phe Val Ala Gly
    130                 135                 140

Ala Asp Asp Glu Ala Ser Asp Ala Lys Asn Tyr Gln Lys Tyr His Glu
145                 150                 155                 160

Gln Ser Arg Asp Ala Phe Leu Lys Tyr Val Lys Asp Asn Glu Ser Thr
                165                 170                 175

Leu Ile Pro Asp Val Phe Lys Asp Gln Glu Asp Val Asn Phe Tyr Ala
            180                 185                 190

Arg Val Ile Glu Asp Lys Ser His Asp Trp Glu Ser Thr Pro Ala His
        195                 200                 205

Val Leu Ile Asn Gln Gly His Met Val Asp Leu Val Arg Val Lys Gln
    210                 215                 220

Pro Pro Glu Ser Phe Leu Gln Arg Tyr Phe Ser Ser Met Gln Arg Trp
225                 230                 235                 240

Ile Gly Ser Gln Ala Thr Glu Ala Val Phe Gly Ile Gln Arg Gln Phe
                245                 250                 255

Phe His Ala Thr Tyr Glu Val Val Ala Gly Phe Asp Ser Asp Asn Lys
            260                 265                 270

Glu Pro His Leu Val Val Ser Gly Leu Gly Arg Tyr Val Ile Gly Glu
        275                 280                 285

Asp Gly Gln Pro Ile Arg Glu Ala Pro Lys Lys Gly Gln Lys Glu Gly
    290                 295                 300

Asp Leu Lys Val Phe Pro Gln Thr Tyr Lys Leu Lys Glu Asn Glu Arg
305                 310                 315                 320

Leu Met Arg Val Asp Glu Phe Leu Lys Leu Pro Glu Ile Gln Asn Thr
                325                 330                 335

Phe Pro Gly Ser Gly Lys His Leu Gln Gly Gly Met Pro Gly Met Asn
            340                 345                 350

Glu Met Asp Tyr Trp Asn Arg Leu Asn Ser Leu Asn Arg Ala Arg Cys
        355                 360                 365

Glu Asn Asp Val Asp Phe Cys Leu Lys Gln Leu Gln Thr Ala His Asp
    370                 375                 380

Lys Ala Lys Ile Glu Pro Ile Lys Gln Ala Phe Gln Ser Ser Lys Gly
385                 390                 395                 400

Lys Glu Arg Arg Gln Pro Asn Val Asp Glu Ile Ala Ala Ala Arg Ile
                405                 410                 415

Ile Gln Gln Ile Leu Ala Asn Pro Asp Cys Ile His Asp Asp His Val
            420                 425                 430

Leu Ile Asn Gly Gln Lys Leu Glu Gln Gln Phe Phe Arg Asp Leu Leu
        435                 440                 445

Ala Lys Cys Glu Met Ala Val Val Gly Ser Leu Leu Asn Asp Thr Asp
    450                 455                 460

Ile Gly Asn Ile Asp Thr Leu Met Arg His Glu Lys Asp Thr Glu Phe
465                 470                 475                 480

His Ser Thr Asn Pro Glu Ala Val Pro Val Lys Ile Gly Glu Tyr Trp
                485                 490                 495

Ile Asn Asp Gln Arg Ile Asn Asn Ser Ser Gly Asn Ile Thr Gln Lys
            500                 505                 510

Lys His Asp Leu Ile Phe Leu Met Gln Asn Asp Ala Trp Tyr Phe Ser
        515                 520                 525

Arg Val Asn Ala Ile Ala Gln Asn Arg Asp Lys Gly Ser Thr Phe Lys
    530                 535                 540

Glu Val Leu Ile Thr Thr Leu Met Thr Pro Leu Thr Ser Lys Ala Leu
545                 550                 555                 560

Val Asp Thr Ser Gln Ala Lys Pro Pro Thr Arg Leu Phe Arg Gly Leu
                565                 570                 575

Asn Leu Ser Glu Glu Phe Thr Lys Gly Leu Ile Asp Gln Ala Asn Ala
            580                 585                 590

Met Ile Ala Asn Thr Thr Glu Arg Leu Phe Thr Asp His Ser Pro Glu
        595                 600                 605

Ala Phe Lys Gln Ile Lys Leu Asn Asp Leu Ser Lys Met Ser Gly Arg
    610                 615                 620

Thr Asn Ala Ser Thr Thr Thr Glu Ile Lys Leu Val Lys Glu Thr Trp
625                 630                 635                 640

Asp Ser Asn Val Ile Phe Glu Met Leu Asp Pro Asp Gly Leu Leu His
                645                 650                 655

Ser Lys Gln Val Gly Arg His Gly Glu Gly Thr Glu Ser Glu Phe Ser
            660                 665                 670

Val Tyr Leu Pro Glu Asp Val Ala Leu Val Pro Val Lys Val Thr Leu
        675                 680                 685

Asp Gly Lys Thr Gln Lys Gly Glu Asn Arg Tyr Val Phe Thr Phe Val
    690                 695                 700

Ala Val Lys Ser Pro Asp Phe Thr Pro Arg His Glu Ser Gly Tyr Ala
705                 710                 715                 720

Val Glu Pro Phe Leu Arg Met Gln Ala Ala Lys Leu Ala Glu Val Lys
                725                 730                 735

Ser Ser Ile Glu Lys Ala Gln Arg Ala Pro Asp Leu Glu Thr Ile Phe
            740                 745                 750

Asn Leu Gln Asn Glu Val Glu Ala Val Gln Tyr Ser His Leu Ser Thr
        755                 760                 765

Gly Tyr Lys Asn Phe Leu Lys Asn Thr Val Gly Pro Val Leu Glu Asn
    770                 775                 780

Ser Leu Ser Gly Leu Met Glu Ser Asp Thr Asp Thr Leu Ser Lys Ala
785                 790                 795                 800

Leu Ala Ala Phe Pro Ser
                805

<210>  6
<211>  806
<212>  PRT
<213>  Legionella pneumophila

<400>  6

Lys Asp Leu Lys Ile Glu Ile Pro Thr Leu Val Leu Pro Gly Lys Asp
1               5                   10                  15

Thr Ser Pro Lys Glu Met Ser Val Lys Pro Thr Ala Pro Gln Asp Lys
            20                  25                  30

Ser Val Pro Val Trp Asn Gly Phe Ser Leu Tyr Thr Asp Asp Thr Val
        35                  40                  45

Lys Ala Ala Ala Gln Tyr Ala Tyr Asp Asn Tyr Leu Gly Lys Pro Tyr
    50                  55                  60

Thr Gly Ser Val Glu Ser Ala Pro Ala Asn Phe Gly Gly Arg Met Val
65                  70                  75                  80

Tyr Arg Gln His His Gly Leu Ser Ala Thr Leu Arg Thr Met Ala Tyr
                85                  90                  95

Ala Glu Leu Ile Val Glu Glu Ala Arg Lys Ala Lys Leu Arg Gly Glu
            100                 105                 110

Thr Leu Gly Lys Phe Lys Asp Gly Arg Thr Ile Ala Asp Val Thr Pro
        115                 120                 125

Gln Glu Leu Lys Lys Ile Met Ile Ala Gln Ala Phe Phe Val Ala Gly
    130                 135                 140

Arg Asp Asp Glu Ala Ser Asp Ala Lys Asn Tyr Gln Lys Tyr His Glu
145                 150                 155                 160

Gln Ser Arg Asp Ala Phe Leu Lys Tyr Val Lys Asp Asn Glu Ser Thr
                165                 170                 175

Leu Ile Pro Asp Val Phe Lys Asp Gln Glu Asp Val Asn Phe Tyr Ala
            180                 185                 190

Arg Val Ile Glu Asp Lys Ser His Asp Trp Glu Ser Thr Pro Ala His
        195                 200                 205

Val Leu Ile Asn Gln Gly His Met Val Asp Leu Val Arg Val Lys Gln
    210                 215                 220

Pro Pro Glu Ser Phe Leu Gln Arg Tyr Phe Ser Ser Met Gln Arg Trp
225                 230                 235                 240

Ile Gly Ser Gln Ala Thr Glu Ala Val Phe Gly Ile Gln Arg Gln Phe
                245                 250                 255

Phe His Ala Thr Tyr Glu Val Val Ala Gly Phe Asp Ser Asp Asn Lys
            260                 265                 270

Glu Pro His Leu Val Val Ser Gly Leu Gly Arg Tyr Val Ile Gly Glu
        275                 280                 285

Asp Gly Gln Pro Ile Arg Glu Ala Pro Lys Lys Gly Gln Lys Glu Gly
    290                 295                 300

Asp Leu Lys Val Phe Pro Gln Thr Tyr Lys Leu Lys Glu Asn Glu Arg
305                 310                 315                 320

Leu Met Arg Val Asp Glu Phe Leu Lys Leu Pro Glu Ile Gln Asn Thr
                325                 330                 335

Phe Pro Gly Ser Gly Lys His Leu Gln Gly Gly Met Pro Gly Met Asn
            340                 345                 350

Glu Met Asp Tyr Trp Asn Arg Leu Asn Ser Leu Asn Arg Ala Arg Cys
        355                 360                 365

Glu Asn Asp Val Asp Phe Cys Leu Lys Gln Leu Gln Thr Ala His Asp
    370                 375                 380

Lys Ala Lys Ile Glu Pro Ile Lys Gln Ala Phe Gln Ser Ser Lys Gly
385                 390                 395                 400

Lys Glu Arg Arg Gln Pro Asn Val Asp Glu Ile Ala Ala Ala Arg Ile
                405                 410                 415

Ile Gln Gln Ile Leu Ala Asn Pro Asp Cys Ile His Asp Asp His Val
            420                 425                 430

Leu Ile Asn Gly Gln Lys Leu Glu Gln Gln Phe Phe Arg Asp Leu Leu
        435                 440                 445

Ala Lys Cys Glu Met Ala Val Val Gly Ser Leu Leu Asn Asp Thr Asp
    450                 455                 460

Ile Gly Asn Ile Asp Thr Leu Met Arg His Glu Lys Asp Thr Glu Phe
465                 470                 475                 480

His Ser Thr Asn Pro Glu Ala Val Pro Val Lys Ile Gly Glu Tyr Trp
                485                 490                 495

Ile Asn Asp Gln Arg Ile Asn Asn Ser Ser Gly Asn Ile Thr Gln Lys
            500                 505                 510

Lys His Asp Leu Ile Phe Leu Met Gln Asn Asp Ala Trp Tyr Phe Ser
        515                 520                 525

Arg Val Asn Ala Ile Ala Gln Asn Arg Asp Lys Gly Ser Thr Phe Lys

530                 535                 540

Glu Val Leu Ile Thr Thr Leu Met Thr Pro Leu Thr Ser Lys Ala Leu
545                 550                 555                 560

Val Asp Thr Ser Gln Ala Lys Pro Pro Thr Arg Leu Phe Arg Gly Leu
                565                 570                 575

Asn Leu Ser Glu Glu Phe Thr Lys Gly Leu Ile Asp Gln Ala Asn Ala
            580                 585                 590

Met Ile Ala Asn Thr Thr Glu Arg Leu Phe Thr Asp His Ser Pro Glu
        595                 600                 605

Ala Phe Lys Gln Ile Lys Leu Asn Asp Leu Ser Lys Met Ser Gly Arg
    610                 615                 620

Thr Asn Ala Ser Thr Thr Thr Glu Ile Lys Leu Val Lys Glu Thr Trp
625                 630                 635                 640

Asp Ser Asn Val Ile Phe Glu Met Leu Asp Pro Asp Gly Leu Leu His
                645                 650                 655

Ser Lys Gln Val Gly Arg His Gly Glu Gly Thr Glu Ser Glu Phe Ser
            660                 665                 670

Val Tyr Leu Pro Glu Asp Val Ala Leu Val Pro Val Lys Val Thr Leu
        675                 680                 685

Asp Gly Lys Thr Gln Lys Gly Glu Asn Arg Tyr Val Phe Thr Phe Val
    690                 695                 700

Ala Val Lys Ser Pro Asp Phe Thr Pro Arg His Glu Ser Gly Tyr Ala
705                 710                 715                 720

Val Glu Pro Phe Leu Arg Met Gln Ala Ala Lys Leu Ala Glu Val Lys
                725                 730                 735

Ser Ser Ile Glu Lys Ala Gln Arg Ala Pro Asp Leu Glu Thr Ile Phe
            740                 745                 750

Asn Leu Gln Asn Glu Val Glu Ala Val Gln Tyr Ser His Leu Ser Thr
        755                 760                 765

Gly Tyr Lys Asn Phe Leu Lys Asn Thr Val Gly Pro Val Leu Glu Asn
    770                 775                 780

Ser Leu Ser Gly Leu Met Glu Ser Asp Thr Asp Thr Leu Ser Lys Ala
785                 790                 795                 800

Leu Ala Ala Phe Pro Ser
                805

<210>  7
<211>  806
<212>  PRT
<213>  Legionella pneumophila

<400>  7

Lys Asp Leu Lys Ile Glu Ile Pro Thr Leu Val Leu Pro Gly Lys Asp
1               5                   10                  15

Thr Ser Pro Lys Glu Met Ser Val Lys Pro Thr Ala Pro Gln Asp Lys
            20                  25                  30

Ser Val Pro Val Trp Asn Gly Phe Ser Leu Tyr Thr Asp Asp Thr Val
        35                  40                  45

Lys Ala Ala Ala Gln Tyr Ala Tyr Asp Asn Tyr Leu Gly Lys Pro Tyr
    50                  55                  60

Thr Gly Ser Val Glu Ser Ala Pro Ala Asn Phe Gly Gly Arg Met Val
65                  70                  75                  80

Tyr Arg Gln His His Gly Leu Ser His Thr Leu Arg Thr Met Ala Tyr
                85                  90                  95

Ala Glu Leu Ile Val Glu Glu Ala Arg Lys Ala Lys Leu Arg Gly Glu
            100                 105                 110

Thr Leu Gly Lys Phe Lys Asp Gly Arg Thr Ile Ala Asp Val Thr Pro
        115                 120                 125

Gln Glu Leu Lys Lys Ile Met Ile Ala Gln Ala Phe Phe Val Ala Gly
    130                 135                 140

Arg Asp Asp Glu Ala Ser Asp Ala Lys Asn Tyr Gln Lys Tyr His Glu
145                 150                 155                 160

Gln Ser Arg Asp Ala Phe Leu Lys Tyr Val Lys Asp Asn Glu Ser Thr
                165                 170                 175

Leu Ile Pro Asp Val Phe Lys Asp Gln Glu Asp Val Asn Phe Tyr Ala
            180                 185                 190

Arg Val Ile Glu Asp Lys Ser His Asp Trp Glu Ser Thr Pro Ala His
        195                 200                 205

Val Leu Ile Asn Gln Gly His Met Val Asp Leu Val Arg Val Lys Gln
    210                 215                 220

Pro Pro Glu Ser Phe Leu Gln Arg Tyr Phe Ser Ser Met Gln Arg Trp
225                 230                 235                 240

Ile Gly Ser Gln Ala Thr Glu Ala Val Phe Gly Ile Gln Arg Gln Phe
                245                 250                 255

Phe His Ala Thr Tyr Glu Val Val Ala Gly Phe Asp Ser Asp Asn Lys
            260                 265                 270

Glu Pro His Leu Val Val Ser Gly Leu Gly Arg Tyr Val Ile Gly Glu
        275                 280                 285

Asp Gly Gln Pro Ile Arg Glu Ala Pro Lys Lys Gly Gln Lys Glu Gly
    290                 295                 300

Asp Leu Lys Val Phe Pro Gln Thr Tyr Lys Leu Lys Glu Asn Glu Arg
305                 310                 315                 320

Leu Met Arg Val Asp Glu Phe Leu Lys Leu Pro Glu Ile Gln Asn Thr
                325                 330                 335

Phe Pro Gly Ser Gly Lys His Leu Gln Gly Gly Met Pro Gly Met Asn
            340                 345                 350

Glu Met Asp Tyr Trp Asn Arg Leu Asn Ser Leu Asn Arg Ala Arg Cys
        355                 360                 365

Glu Asn Asp Val Asp Phe Cys Leu Lys Gln Leu Gln Thr Ala His Asp
    370                 375                 380

Lys Ala Lys Ile Glu Pro Ile Lys Gln Ala Phe Gln Ser Ser Lys Gly
385                 390                 395                 400

Lys Glu Arg Arg Gln Pro Asn Val Asp Glu Ile Ala Ala Ala Arg Ile
                405                 410                 415

Ile Gln Gln Ile Leu Ala Asn Pro Asp Cys Ile His Asp Asp His Val
            420                 425                 430

Leu Ile Asn Gly Gln Lys Leu Glu Gln Gln Phe Phe Arg Asp Leu Leu
        435                 440                 445

Ala Lys Cys Glu Met Ala Val Val Gly Ser Leu Leu Asn Asp Thr Asp
    450                 455                 460

Ile Gly Asn Ile Asp Thr Leu Met Arg His Glu Lys Asp Thr Glu Phe
465                 470                 475                 480

His Ser Thr Asn Pro Glu Ala Val Pro Val Lys Ile Gly Glu Tyr Trp
                485                 490                 495

Ile Asn Asp Gln Arg Ile Asn Asn Ser Ser Gly Asn Ile Thr Gln Lys
            500                 505                 510

Lys His Asp Leu Ile Phe Leu Met Gln Asn Asp Ala Trp Tyr Phe Ser
        515                 520                 525

Arg Val Asn Ala Ile Ala Gln Asn Arg Asp Lys Gly Ser Thr Phe Lys
    530                 535                 540

Glu Val Leu Ile Thr Thr Leu Met Thr Pro Leu Thr Ser Lys Ala Leu
545                 550                 555                 560

Val Asp Thr Ser Gln Ala Lys Pro Pro Thr Arg Leu Phe Arg Gly Leu
                565                 570                 575

Asn Leu Ser Glu Glu Phe Thr Lys Gly Leu Ile Asp Gln Ala Asn Ala
            580                 585                 590

Met Ile Ala Asn Thr Thr Glu Arg Leu Phe Thr Asp His Ser Pro Glu
        595                 600                 605

Ala Phe Lys Gln Ile Lys Leu Asn Asp Leu Ser Lys Met Ser Gly Arg
    610                 615                 620

Thr Asn Ala Ser Thr Thr Thr Glu Ile Lys Leu Val Lys Glu Thr Trp
625                 630                 635                 640

Asp Ser Asn Val Ile Phe Glu Met Leu Asp Pro Asp Gly Leu Leu His
                645                 650                 655

Ser Lys Gln Val Gly Arg His Gly Glu Gly Thr Ala Ser Ala Phe Ser
            660                 665                 670

Val Tyr Leu Pro Glu Asp Val Ala Leu Val Pro Val Lys Val Thr Leu
        675                 680                 685

Asp Gly Lys Thr Gln Lys Gly Glu Asn Arg Tyr Val Phe Thr Phe Val
    690                 695                 700

Ala Val Lys Ser Pro Asp Phe Thr Pro Arg His Glu Ser Gly Tyr Ala
705                 710                 715                 720

Val Glu Pro Phe Leu Arg Met Gln Ala Ala Lys Leu Ala Glu Val Lys
                725                 730                 735

Ser Ser Ile Glu Lys Ala Gln Arg Ala Pro Asp Leu Glu Thr Ile Phe
            740                 745                 750

Asn Leu Gln Asn Glu Val Glu Ala Val Gln Tyr Ser His Leu Ser Thr
        755                 760                 765

Gly Tyr Lys Asn Phe Leu Lys Asn Thr Val Gly Pro Val Leu Glu Asn
    770                 775                 780

Ser Leu Ser Gly Leu Met Glu Ser Asp Thr Asp Thr Leu Ser Lys Ala
785                 790                 795                 800

Leu Ala Ala Phe Pro Ser
                805

## Description

### FIELD OF THE INVENTION

The present invention pertains to the modification of the eukaryotic ubiquitin system by using the bacterial virulence factor SdeA and variants and mutants thereof The invention is based on the phosphodiesterase activity of SdeA, which catalyzes a phospho-ribosylation (or simply "ribosylation") event of ubiquitin and ubiquitin-like proteins. The invention provides SdeA proteins and SdeA mutants without said phosphodiesterase activity, as well as medical applications of these recombinant proteins, their encoding nucleic acids, ribosylated ubiquitin proteins, and compounds which may act as selective or non-selective phosphodiesterase inhibitors.

### DESCRIPTION

The present invention pertains to a method for modulating the cellular ubiquitin system. The ubiquitin system is involved in regulating almost every cellular pathway and thus plays a critical role in many human diseases. Inhibiting the ubiquitin system is valuable in studying a protein's function that is prone to degradation in cells and also in treating diseases where the ubiquitin system is upregulated or misregulated. The ubiquitination cascade starts with ubiquitin activation by a ubiquitin-activating enzyme (E1), which forms a high energy thioester linkage with the C-terminus of ubiquitin in the presence of ATP as the energy source. E1 interacts with a ubiquitin-conjugating enzyme (E2) and ubiquitin is transferred to the E2 via a trans-thioesterification reaction. Finally, with the help of a ubiquitin ligase (E3), ubiquitin is typically attached to a lysine residue on a protein substrate, forming an isopeptide bond via its C-terminus. Depending on the type of modification (mono-, poly-, or multi-ubiquitination), the modified substrate protein can be targeted for proteasomal degradation, or can change its cellular localization, interaction partners or enzymatic function.

The main cellular machinery for the degradation of intracellular proteins is the ubiquitin-proteasome system. Substrates tagged with a poly-ubiquitin tail are marked for degradation by the proteasome. In eukaryotes, proteins are constantly synthesized and degraded, the configuration of the proteasome is responsible for the degradation of proteins into short peptide fragments. Because of the enormous destructive potential of the proteasome, both its localization and activity in a cell is under tight control. Only proteins that are marked with a covalently linked poly-ubiquitin tail of at least 4 mono-ubiquitin molecules in length can enter the proteasome and are subsequently degraded into short peptide fragments. Not all proteins require poly-ubiquitination before proteasomal degradation. One notable exception is ornithine decarboxylase (ODC) which activates its degradation by the proteasome via a direct interaction with a regulatory subunit.

Because of the involvement of the proteasome in many important cellular processes it has become a target of interest in the search of novel therapies for a wide variety of diseases. For instance, proteasome inhibitors have already proven themselves to be of great therapeutic value as illustrated by the approval of Velcade® (bortezomib) for the treatment of multiple myeloma and mantle cell lymphoma. In contrast to proteasome inhibition, proteasome activation is a relatively new field. Increasing the proteolytic capacity of cells could have potential therapeutic applications for the treatment of e.g. neurodegenerative diseases. The identification of proteasome modulators and the study of their regulating dynamics will contribute to the general knowledge about proteasome activity and assist in the development of new therapies for a variety of diseases.

Given the essential roles of ubiquitination in the regulation of the immune system, it is not surprising that the ubiquitination network is a common target for diverse infectious agents. For example, many bacterial pathogens exploit ubiquitin signaling using virulence factors that function as E3 ligases, deubiquitinases or as enzymes that directly attack ubiquitin. The bacterial pathogen *Legionella pneumophila* utilizes approximately 300 effectors that modulate diverse host processes to create a permissive niche for its replication in phagocytes.

Recently it was demonstrated that a member of the SidE effector family of *L. pneumophila,* the protein SdeA, ubiquitinates multiple Rab small GTPases associated with the endoplasmic reticulum without the need for the E1 and E2 enzymes (Qui J et al., "Ubiquitination independent of E1 and E2 enzymes by bacterial effectors."; Nature. 2016 May 5;533(7601):120-4. doi: 10.1038/nature17657. Epub 2016 Apr 6.). This E1/E2-independent ubiquitination catalyzed by SdeA is energized by nicotinamide adenine dinucleotide, which activates ubiquitin by the formation of ADP-ribosylated ubiquitin. These results establish that ubiquitination can be catalyzed by a single enzyme, the activity of which does not require ATP.

In view of the state of the art it was an object of the invention to provide novel approaches for the modulation, in particular the impairment, of the ubiquitin-proteasome system in eukaryotes. Furthermore the invention seeks to provide compounds usable in methods for the modulation of the ubiquitin system and the application of these compounds in the treatment of the various ubiquitin- and proteasome-associated disorders.

The above problem is solved in a first aspect by a method of deactivating, blocking and/or impairing the ubiquitin system in a cell, comprising introducing into the cell a recombinant SdeA protein, or a recombinant SdeA protein derivative, wherein the SdeA protein, or the recombinant SdeA protein derivative, comprises at least an ADP-ribosyl transferase domain.

In context of the present invention a "SdeA protein" is preferably a protein having the amino acid sequence as shown in SEQ ID NO: 1. SdeA in context of the invention shall be understood to be a protein comprising at least the enzymatic domains: deubiquitinating protease (DUB), phosphodiesterase domain and a ADP-ribosyltransferase domain. In one alternative embodiment the SdeA protein of the invention is a protein, comprising an amino acid sequence having at least 80%, preferably 85%, 90%, 95%, 96%, 97%, most preferably 98% or 99% sequence identity to the sequence shown in SEQ ID NO: 1.

In some embodiments it is preferable that the SdeA protein used in the methods of the invention is a recombinant SdeA protein, or a recombinant SdeA protein derivative, which does not comprise a functional phosphodiesterase domain. A SdeA protein not having a functional phosphodiesterase domain is preferably a mutated SdeA, such as mutant SdeA proteins with alterations in the amino acid sequence of the phosphodiesterase domain. These alterations are in some embodiments selected from mutations at residues corresponding to H284 and/or R344 in SEQ ID NO: 1. Preferably, the mutations are H284A, and/or R344A, respectively. In other embodiments mutants may be selected from any deletion, substitution, inversion, or insertion of an amino acid sequence of SdeA which in consequence will reduce or diminish the phosphodiesterase activity of the protein. The person of skill may for example screen for various mutant SdeA proteins using the assays described in the example section of this application. In the following such a protein variant of SdeA is also referred to as a "mutant SdeA" protein.

In alternative aspect, the above problem of the prior art is also solved by a mutant SdeA protein as described herein above.

In some other embodiments it is preferable that the recombinant SdeA protein, or the recombinant SdeA protein derivative, of the invention comprises at least an ADP-ribosyl transferase domain, and does not comprise a functional phosphodiesterase domain.

A mutated SdeA protein according to the present descriptions comprises preferably an amino acid sequence that is at least 50 %, 60%, 70%, 80%, 90%, or most preferably 95% identical to a sequence selected from SEQ ID NOs: 1 to 7. A "wild-type SdeA" is in context of the invention an SdeA protein having a sequence occurring in a wild organism. A "mutated SdeA" comprises a sequence which is not wild-type, and therefore mutated, for example by the artificial introduction of a sequence alteration into the wild type sequence.

In some embodiments the method of the invention may comprise an additional step of contacting the cell with NAD, in particular in the event that NAD is not present in sufficient amounts to allow for the ribosylation reaction to occur.

A cell in context of the invention wherein the ubiquitin system is to be modified is preferably a eukaryotic cell, preferably a mammalian or human cell.

The herein described methods are preferably in some embodiments performed *in vitro* or *ex vivo.*

In context of the invention the recombinant SdeA protein, or the recombinant SdeA protein derivative, is introduced into the cell either directly or by using a nucleic acid encoding the recombinant SdeA protein, or the recombinant SdeA protein derivative. Such nucleic acids are preferably introduced into a biological cell via transfection or transduction, for example using a retrovirus or lentivirus, of an expression construct encoding the recombinant SdeA protein, or the recombinant SdeA protein derivative.

Furthermore provided is a nucleic acid construct, comprising a nucleic acid sequence encoding the mutated SdeA protein as described herein elsewhere. A nucleic acid construct of the invention is preferably a vector, such as an expression vector comprising a promoter sequence operably linked to the sequence encoding the mutated SdeA protein.

A variety of methods and systems for DNA delivery into a biological cell are known in the art and may be used within the compositions and methods of the presently disclosed subject matter, including without limitation lipid-based systems (e.g., cationic lipids, lipoplexes, lipid-modified polycations, emulsions, and high density lipoprotein (HDL)); organic nanoparticles (e.g., polyplexes, micelles (block and graft copolymers), and nanogels); inorganic nanoparticles (e.g., calcium phosphate particles, super paramagnetic iron oxide nanoparticles (SPIONs) and nanodiamonds); and natural vesicles (e.g., virus like particles (VLPs) and exosomes).

Vectors which comprise the nucleic acid constructs described herein are also encompassed by embodiments of the invention and include both viral and non-viral vectors. As used herein, the term "vector" refers to a nucleic acid construct designed for transduction/transfection of one or more cell types. Vectors may be, for example, "cloning vectors," which are designed for isolation, propagation and replication of inserted nucleotides, "expression vectors," which are designed for expression of a nucleotide sequence in a host cell, a "viral vector," which is designed to result in the production of a recombinant virus or virus-like particle, or "shuttle vectors," which comprise the attributes of more than one type of vector. The term "replication" means duplication of a vector.

The term "expression vector" is used interchangeably herein with the term "plasmid" and "vector" and refers to a recombinant DNA molecule containing a desired coding sequence and appropriate nucleic acid sequences necessary for expression of the operably linked coding sequence (e.g., an insert sequence that codes for a product) in a particular host cell. The term "plasmid" refers to an extrachromosomal circular DNA capable of autonomous replication in a given cell. In certain embodiments, the plasmid is designed for amplification and expression in bacteria. Plasmids can be engineered by standard molecular biology techniques. See Sambrook et al, Laboratory Manual, Cold Spring Harbor Laboratory Press, Cold Spring Harbor (1989), N.Y. Nucleic acid sequences necessary for expression in prokaryotes usually include a promoter, an operator (optional), and a ribosome binding site, often along with other sequences.

Exemplary viral vectors include but are not limited to: bacteriophages, various baculoviruses, retroviruses, and the like. Those of skill in the art are familiar with viral vectors that are used in gene therapy applications, which include but are not limited to: Herpes simplex virus vectors (Geller et al. (1988) Science 241 : 1667-1669); vaccinia virus vectors (Piccini et al. (1987) Meth. Enzymology, 153 :545-563); cytomegalovirus vectors (Mocarski et al, in Viral Vectors, Y. Gluzman and S. H. Hughes, Eds., Cold Spring Harbor Laboratory, Cold Spring Harbor, N.Y., 1988, pp. 78-84)); Moloney murine leukemia virus vectors (Danos et al. (1988) Proc. Natl. Acad. Sci. USA 85:6460-6464; Blaese et al. (1995) Science 270:475-479; Onodera et al. (1988) J. Virol. 72: 1 '69-17'4; adenovirus vectors (Berkner (1988) Biotechniques 6:616-626; Cotten et al. (1992) Proc. Natl. Acad. Sci. USA 89:6094-6098; Graham et al. (1991) Meth. Mol. Biol. 7: 109-127; Li et al. (1993) Human Gene Therapy 4:403- 409; Zabner et al. (1994) Nature Genetics 6:75-83); adeno-associated virus vectors (Goldman et al. (1997) Human Gene Therapy 10:2261-2268; Greelish et al. (1999) Nature Med. 5:439-443; Wang et al. (1999) Proc. Natl. Acad. Sci. USA 96:3906-3910; Snyder et al. (1999) Nature Med. 5:64-70; Herzog et al. (1999) Nature Med. 5:56-63); retrovirus vectors (Donahue et al. (1998) Nature Med. 4: 181-186; Shackleford et al. (1988) Proc. Natl. Acad. Sci. USA 85:9655-9659; U.S. Pat. Nos. 4,405,712, 4,650,764 and 5,252,479, and PCT Patent Publication Nos. WO 92/07573, WO 90/06997, WO 89/05345, WO 92/05266 and WO 92/14829; and lentivirus vectors (Kafri et al. (1997) Nature Genetics 17:314-317), as well as viruses that are replication-competent conditional to a cancer cell such as oncolytic herpes virus NV 1066 and vaccinia virus GLV-1h68, as described in U.S. Patent Application Pub. No. 2009/031 1664. In particular, adenoviral vectors may be used, e.g. targeted viral vectors such as those described in U.S. Patent Application Pub. No. 2008/0213220. Accordingly, in some aspects, a viral vector comprises the nucleic acid construct wherein the viral vector is selected from the group consisting of adenoviral, lentiviral, retroviral, adeno- associated viral, and herpes simplex viral.

Exemplary non-viral vectors that may be employed include but are not limited to, for example: cosmids or plasmids; and, particularly for cloning large nucleic acid molecules, bacterial artificial chromosome vectors (BACs) and yeast artificial chromosome vectors (YACs); as well as liposomes (including targeted liposomes); cationic polymers; ligand-conjugated lipoplexes; polymer-DNA complexes; poly-L-lysine-molossin-DNA complexes; chitosan-DNA nanoparticles; polyethylenimine (PEI, e.g. branched PEI)-DNA complexes; various nanoparticles and/or nanoshells such as multifunctional nanoparticles, metallic nanoparticles or shells (e.g. positively, negatively or neutral charged gold particles, cadmium selenide, etc.); ultrasound- mediated microbubble delivery systems; various dendrimers (e.g. polyphenylene and poly(amidoamine)-based dendrimers; etc. In still further aspects, the non-viral vector is a nanoparticle is selected from the group consisting of: a liposome, an exosome, a nanodiamond, a polyphosphazene, a dendrimer, a polyplex, a lipoplex, a polymeric nanoconjugate, a high density lipoprotein (HDL), a fluorescent super paramagnetic iron oxide nanoparticle (FSPION), a gel (e.g., chitosan or gelatin), a block copolymer micelle, and an inversion emulsion. Exemplary polyplexes include, e.g., a polyethylenimine (PEI), a polypropylenimine (PPI), a poly-L-lysine (PLL), a Poly(amidoamine) (PAMAM), and a poly(2-dimethylaminoethyl methacrylate) (PDMAEMA). Exemplary lipoplexes include, e.g., 1,2-di-0-octadecenyl-3- trimethylammonium propane (DOTMA), 1,2-dioleoyl-3-trimethylammonium-propane (DOTAP), 1,2-dimyristy-loxypropyl-3-dimethyl-hydroxyethyl ammonium bromide (DMRIE), 3-[N-(',N'-dimethylaminoethane)-carbamoyl]cholesterol hydrochloride (DC-Choi), Lipdi 67, dioctadecylaminoglycylcarboxyspermine (DOGS), and 2,3-dioleyloxy-N-[2(sperminecarboxamido) ethyl]-N,N-dimethyl-1-propanaminium trifluoroacetate (DOSPA).

Those of skill in the art will recognize that the choice of a particular vector will depend on its precise usage. Typically, one would not use a vector that integrates into the host cell genome due to the risk of insertional mutagenesis, and one should design vectors so as to avoid or minimize the occurrence of recombination within a vector's nucleic acid sequence or between vectors.

Accordingly, in some aspects, the nucleic acid construct is selected from the group consisting of adenoviral, lentiviral, retroviral, adeno-associated viral, and herpes simplex viral.

Host cells which contain the constructs and vectors of the presently disclosed subject matter are also encompassed, e.g. *in vitro* cells such as cultured cells, or bacterial or insect cells which are used to store, generate or manipulate the vectors, and the like. Accordingly, in yet another aspect, cells, particularly cancer cells, comprising the SdeA constructs are also provided. The constructs and vectors may be produced using recombinant technology or by synthetic means.

In yet another aspect of the invention there is provided a method of deactivating, blocking or impairing the ubiquitin system in a cell, comprising contacting the cell with one of (i) a phosphoribosylated ubiquitin, or (ii) an ADP-ribosylated ubiquitin. Surprisingly, the invention discovered the inhibitory effects of the products of the SdeA catalyzed ubiquitin modification. Therefore, the invention developed a method for the targeted inhibition of the ubiquitin system in a cell.

In yet another aspect of the invention there is provided a method of modifying ubiquitin, or ubiquitin-like protein, comprising the step of contacting the ubiquitin, or ubiquitin-like protein, with an SdeA protein or a mutated SdeA protein as described herein before, preferably in the presence of a sufficient amount of NAD. The modification is in accordance with the invention preferably a ribosylation of ubiquitin, or ubiquitin-like protein, or most preferably the ADP-ribosylation of ubiquitin, or ubiquitin-like protein.

The term "ubiquitin-like protein" refers to molecules other than ubiquitin, which confer similar modes of functional protein modification and are henceforth called ubiquitin- like proteins, molecules, or modifiers (ULMs). The family heritage of ubiquitin-like protein modifiers (ULMs) is not so much by sequence homology but rather by a common 3D structure, the ubiquitin fold, and a C-terminal glycine residue, whose carboxyl group is the site of attachment to the lysine residue of substrates via isopeptide bond formation. Hence, they are conjugated to proteins and function in "ubiquitin-like" manner. At least 10 different ULMs exist in mammals. Examples can include, but are not limited to Interferon-induced 17 kDa protein, ISG15 (UCRP), UniProt P05161 (2 ubiquitins); FUB1 (MNSFp), UniProt P35544; NEDD8 (Rubl), UniProt Q15843; FAT 10 (2 ubiquitins), Ubiquitin D; Small ubiquitin-related modifier 1, SUMO-1 (SMT3C, GMP1, UBL1), UniProt P63165; Small ubiquitin-related modifier 2, SUMO-2 (SMT3B), UniProt P61956; Small ubiquitin-related modifier 3, SUMO-3 (SMT3A), UniProt P55854; Autophagy protein 8, Apg 8, LC3 Antibody;LC3A, LC3B, LC3C, Autophagy protein 12, Apg 12; Ubiquitin-related modifier- 1, Urml ; Ubiquitin-like protein 5, UBL5 (Hubl), UniProt Q9BZL1; and Ubiquitin- fold modifier 1, Ufml, UniProt P61960; GABARAP, GABARAPL1, and GABARAPL2.

Furthermore provided is the medical application of the compounds of the invention. Therefore another aspect relates to a compound for use in medicine, wherein the compound is a mutated SdeA protein according to the various descriptions of the invention, a nucleic acid construct as described before, a host cell as described before, or a ribosylated or ADP-ribosylated ubiquitin.

The medical applications of the present invention are preferably the treatment of a tumor disease, or a disease associated with a pathological ubiquitination, proteasomal degradation, mitophagy, autophagy or TNF mediated cell signaling. Various pathologies are known to benefit from a targeted modification of the ubiquitin system. These disorders are for example reviewed in "The ubiquitin system, disease, and drug discovery" by Matthew D Petroski (BMC Biochemistry 2008, 9(Suppl 1):S7). In particular preferred are disorders associated with the function of the proteasome, for which ubiquitination is the major targeting signal.

A variety of conditions or diseases have been known or believed to be mediated by the catalytic functions of the proteasome. Proteasome inhibition has been suggested as a prevention and/or treatment of a multitude of diseases including, but not limited to, cancers, neurotoxic/degenerative diseases, Alzheimer's disease, ischemic conditions, inflammation, immune-related diseases, HIV infection, organ graft rejection, septic shock, inhibition of antigen presentation, parasitic infections, conditions associated with acidosis, macular degeneration, pulmonary conditions, muscle wasting diseases, fibrotic diseases, bone and hair growth diseases.

Proteasome/Ubiquitination inhibition has been clinically demonstrated as an antitumor therapeutic strategy. Accordingly, the compounds disclosed herein are useful for treating cancers. Exemplary cancers that may be treated include leukemia, lymphoma, myeloma, and carcinomas, such as hepatocellular carcinoma etc. Other cancers that may be treated with the compounds disclosed herein include adrenocortical carcinoma, AIDS-related carcinomas, astrocy-toma, bone carcinoma, osteosarcoma, GBM, malignant fibrous histiocytoma, melanoma, malignant mesothelioma, Pheochromocytoma, pineoblastoma and supratentorial primitive neuroectodermal tumors, neuroblastoma, uterine sarcoma, bile duct cancer, bladder cancer, breast cancers, gastrointestinal cancers, cervical cancers, colon cancers, rectal cancers, esophageal cancers, eye cancers, ovarian cancer, head and neck cancers, kidney cancers, lip and oral cavity cancers, lung cancers, nasal cavity and paranasal sinus cancers, penile cancers, prostate cancers, transitional cell cancers, salivary gland cancers, soft tissue cancers, skin cancers, thyroid, parathyroid cancer, and vaginal cancers.

Proteasome/Ubiquitination inhibitors have been associated with NF-κB inhibition. NF-κB is a potent transcription factor which mediates transcription of genes including inflammatory molecules such as TNF, IL-1, cyclooxygenase, ICAM, and etc. Accordingly, the compounds provided herein can be used as an immunosuppressant which may be useful in inflammatory disorders or conditions, such as, without limitation, allergy, asthma, rejection of a transplanted organ/tissue, auto-immune diseases lupus, rheumatoid arthritis, psoriasis, multiple sclerosis, and inflammatory bowel diseases. The compounds provided herein may be administered in an effective amount, optionally in a pharmaceutical composition, to a subject in need thereof, to treat these conditions.

Proteasome/Ubiquitination inhibitors have been found to reduce degradation of muscle proteins, therefore useful in inhibiting muscle loss and fiber atrophy. Accordingly, the compounds provided herein can be used to treat cachexia and muscle-wasting diseases, such as chronic infectious diseases, fever, muscle disuse and denervation, nerve injury, renal failure associated with acidosis, and hepatic failure. In certain embodiments, the compounds provided herein can be administered in an effective amount, optionally in a pharmaceutical composition, to a subject in need thereof, to slow down or reduce muscle protein degradation, intracellular protein degradation or p53 protein degradation in a cell.

The compounds provided herein can also be used to treat neurodegenerative diseases and conditions, including, but not limited to, stroke, ischemic damage to the nervous system, neural trauma (e.g. percussive brain damage, spinal cord injury, and traumatic damage to the nervous system), multiple sclerosis and other immune-mediated neuropathies (e.g. Guillain-Barre syndrome and its variants, acute motor axonal neuropathy, acute inflammatory demyelinating polyneuropathy, and Fisher Syndrome), HIV/AIDS dementia complex, axonomy, diabetic neuropathy, Parkinsons's disease, Huntington's disease, multiple sclerosis, bacterial, parasitic, fungal, and viral meningitis, encephalitis, vascular dementia, multi-infarct dementia, Lewy body dementia, frontal lobe dementia such as Pick's disease, subcortical dementias (such as Huntington or progressive supranuclear palsy), focal cortical atrophy syndromes (such as primary aphasia), metabolic-toxic dementias, and dementias caused by infections (such as syphilis or chronic meningitis). [0073] The compounds provided herein can further be useful in regulating protein processing that is associated with extracellular deposition of β-amyloid protein (β-AP), the major cause of Alzheimer's disease. Accordingly, the compounds provided herein, such as the mutant SdeA proteins, are useful in treating Alzheimer's disease, for example by reducing the rate of β-AP processing, reducing the rate β-AP plaque formation, reducing the rate of β-AP generation, and reducing the clinical symptoms of Alzheimer's disease.

Proteasome/Ubiquitination inhibition has also been further associated with reducing fibrosis. Accordingly, the compounds provided herein can be used for treatment of fibrosis related conditions such as, diabetic nephropathy, glomerulosclerosis, IgA nephropathy, cirrhosis, biliary atresia, congestive heart failure, scleroderma, radiation-induced fibrosis, lung fibrosis, and cardiac fibrosis.

Another aspect of the invention further pertains to a method for identifying a modulator of ubiquitination, comprising a step of contacting a ubiquitin protein with a candidate SdeA variant protein in the presence of NAD and subsequently determining the level of ADP-ribosylation and/or phospho-ribosylation of said ubiquitin protein.

A candidate SdeA variant protein of the invention has preferably a sequence that is at least 50%, 60%, 70%, 80%, 85%, 90%, 95%, and most preferably 98% identical to the sequence selected from SEQ ID NO: 1 to 7.

Another embodiment also pertains to phosphodiesterase inhibitor for use in the treatment of an infectious disease. In context of the invention it was for the first time found that SdeA, a microbiological virulence factor, needs a phosphodiesterase function in order to mediate its effects on the host cells.

A phosphodiesterase (PDE) inhibitor for use according to the invention is preferably a compound that is capable of inhibiting or reducing selectively or non-selectively the activity of a phosphodiesterase, most preferably of the phosphodiesterase activity of SdeA.

A non-selective inhibitor of PDE is selected from the group consisting of caffeine, aminophylline, IBMX (3-isobutyl-1-methylxanthine), paraxanthine, pentoxifylline, theo-bromine, theophylline, and methylated xanthines.

A phosphodiesterarse inhibitor may also be selected from the group consisting of AN2728, Adibendan, Aminophylline, Aminophylline dihydrate, Amipizone, Arofylline, Ati-zoram, Befuraline, Bemarinone hydrochloride, Bemoradan, Benafentrine, Bucladesine, Buflomedil, Buquineran, CC-1088, Carbazeran, Catramilast, Cilomilast, Cilostamide, Cilostazol, Cipam-fylline, Daxalipram, Denbufylline, Dimabefylline, Diniprofylline, Dipyridamole, Doxofylline, Drotaverine, Dyphylline, Enoximone, Etamiphyllin, Etofylline, Filaminast, Flufylline, Fluprofylline, Furafylline, Imazodan, Imazodan hydrochloride, Inamrinone, Inamrinone lactate, Isbufylline, Lirimilast, Lisofylline, Lomifylline, Medorinone, Metescufylline, Midaxifylline, Milrinone, Milrinone lactate, Motapizone, Nanterinone, Nestifylline, Nitraquazone, Oglemilast, Oglemilast Sodium, Olprinone, Oxagrelate, Oxtriphylline, Papaverine, Papaverine hydrochloride, Papaverine sulfate, Parogrelil, Pelrinone hydrochloride, Pentifylline, Pentoxifylline, Perbufylline, Piclamilast, Pimefylline, Pimobendan, Piroximone, Prinoxodan, Proxy-phylline, Pumafentrine, Quazinone, Quazodine, Revamilast, Revizinone, Roflumilast, Rolipram, Ronomilast, Saterinone, Senazodan, Siguazodan, Tetomilast, Tofimilast, Trapidil, Vesnarinone, Aminotadalafil, Avanafil, Beminafil, Dasantafil, Gisadenafil, Gisadenafil besylate, Mirodenafil, Sildenafil, Sildenafil citrate, Tadalafil, Udenafil, Vardenafil, Vardenafil dihydrochloride, Vardenafil hydrochloride trihydrate, Zaprinast and Zardaverine.

A further aspect of the invention pertains to a method for screening for anti-infectious compounds, the method comprising the steps of bringing a candidate compound into contact with a SdeA1 protein and a di-phosphate substrate under conditions that allow for an enzymatically catalyzed hydrolysis of the diphosphate substrate, and comparing the level of hydrolysis of the di-phosphate substrate in the presence of the candidate compound with a control, wherein a reduced level of hydrolysis indicates that the compound is an anti-infectious compound.

A diphosphate substrate of the invention is preferably an ADP-ribosylated substrate, such as preferably an ADP-ribosylated ubiquitin.

The level of hydrolysis may be determined by quantifying the level of released AMP from the reaction.

Preferably the screening method for anti-infectious compounds of the invention further comprises bringing into contact the SdeA protein, NAD and a SdeA-substrate, under conditions that allow for an enzymatic catalyzed ADP phospho-ribosylation of the SdeA-substrate. The SdeA-substrate is selected preferably from ubiquitin and ubiquitin like molecules such as LC3A, LC3B, LC3C, GABARAP, GABARAPL1, GABARAPL2, Sumo-1, Sumo-2, Sumo-3, NEDD8, Ufm1, FAT10 and ISG15.

The present invention will now be further described in the following examples with reference to the accompanying figures and sequences, nevertheless, without being limited thereto. For the purposes of the present invention, all references as cited herein are incorporated by reference in their entireties. In the Figures:

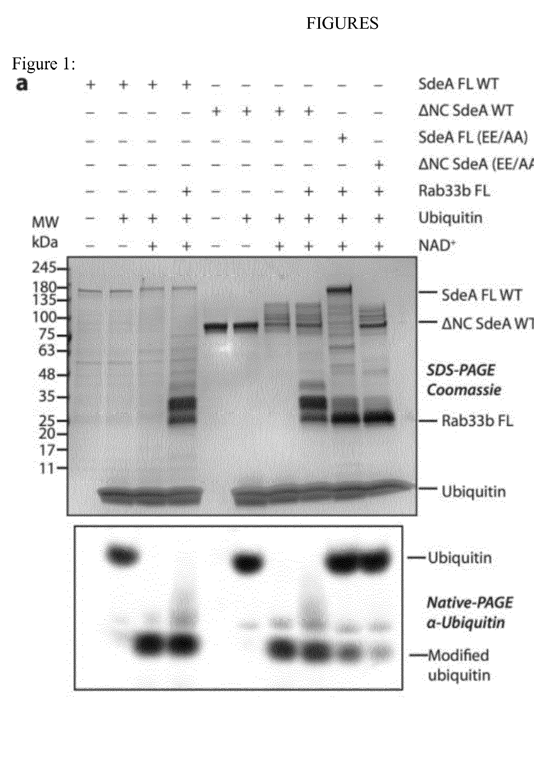

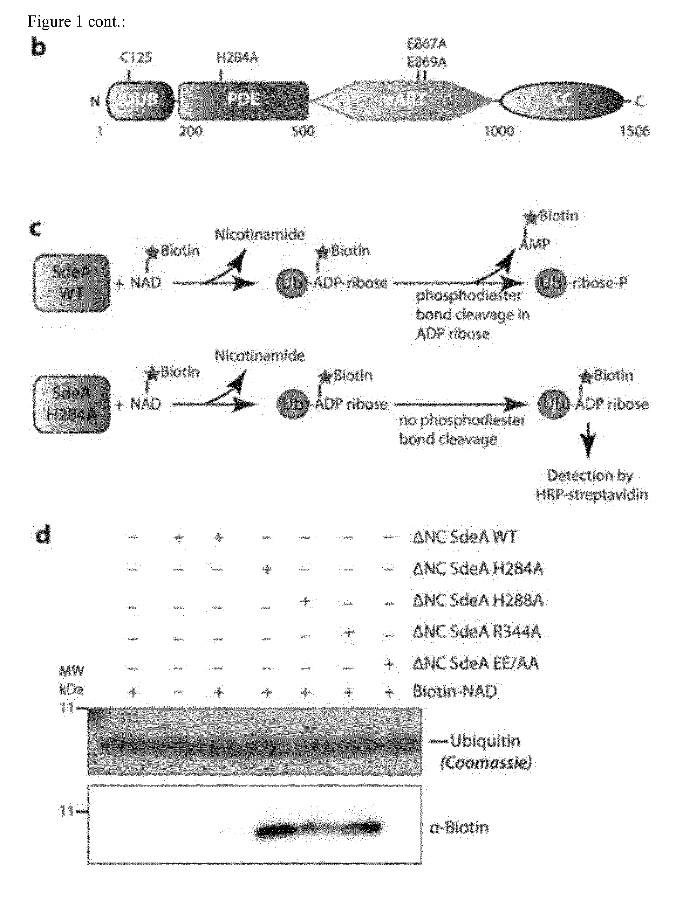

- **Figure 1:**: **Modification of ubiquitin by SdeA wildtype and PDE mutants; A**, SdeA plus NAD⁺ is incubated with excess amounts of ubiquitin in the presence or absence of Rab33b. The reaction mixture was subjected to SDS-PAGE and native-PAGE. Ubiquitin modification can be clearly monitored through the altered electrophoretic mobility of modified ubiquitin in native-PAGE. SdeA modifies the entire ubiquitin pool that is present in many fold excess of substrate. **B,** Schematic representation of domain organization in SdeA. Domain abbreviations are DUB, Deubiquitinase; PDE, Phosphodiesterase; mART, Mono-ADP ribosyl transferase and CC, coiled-coil. The putative catalytic residues in each domain are also shown. **C,** Experimental scheme showing the usage of biotin labeled NAD to probe cleavage of phosphodiester bond in ADP ribosylated ubiquitin; **D,** Testing the function of PDE domain of SdeA using the scheme shown in Fig. 1c. PDE mutants H284, H288 and R344 were defective in phosphodiester bond cleavage and showed positive signal for biotin on ubiquitin.

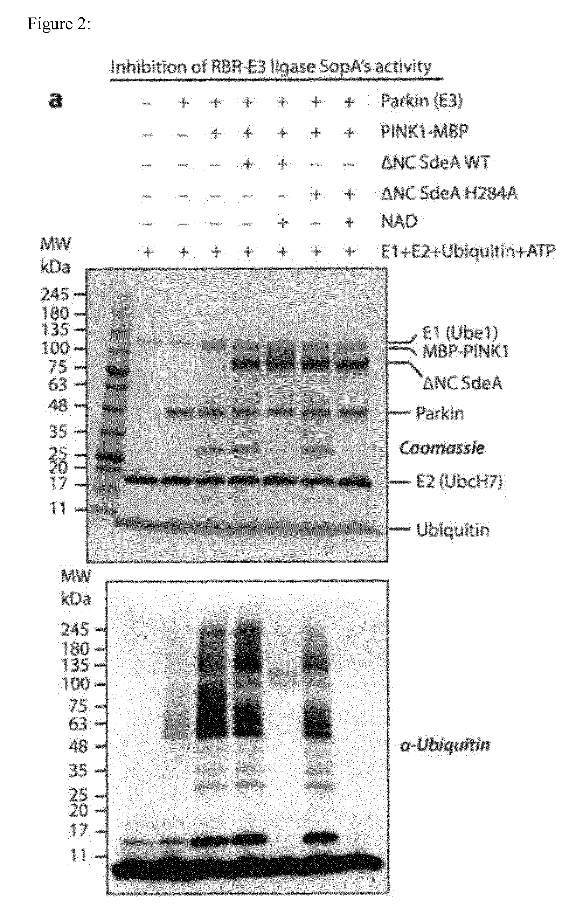

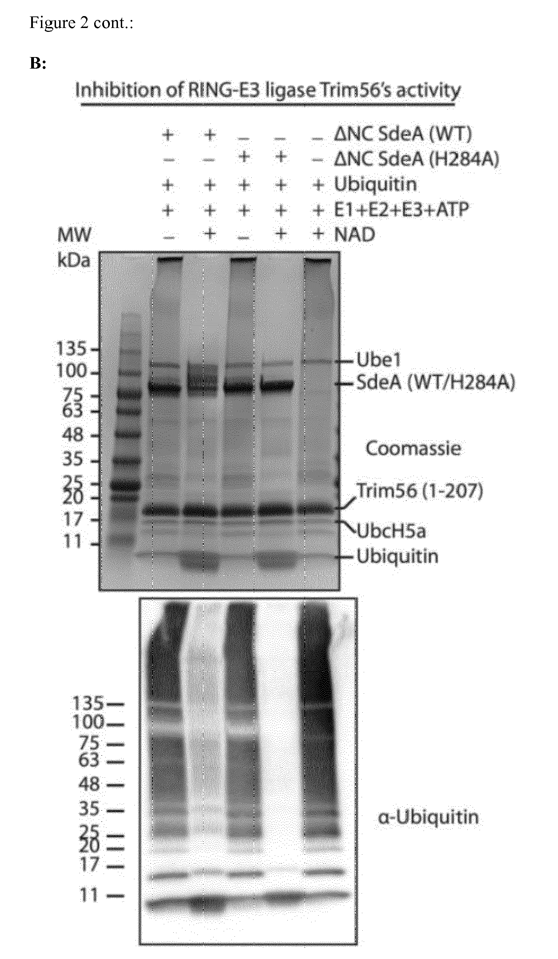

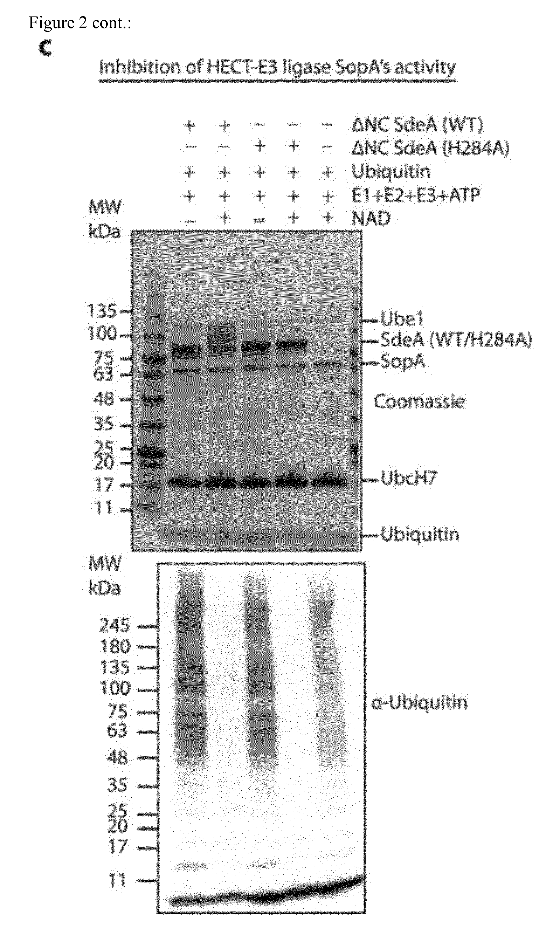

- **Figure 2:**: **Modification of ubiquitin by SdeA inhibits ubiquitination by RBR, HECT and RING type E3 ligases A**, Activity of Parkin was tested in an *in vitro* ubiquitination assay using either wildtype ubiquitin or SdeA-modified ubiquitin. Both phosphoribosylated ubiquitin (produced by treatment with wildtype SdeA) and the ADP-ribosylated ubiquitin (produced by treatment with H284A SdeA) were defective in polyubiquitin chain formation in these reactions as seen by both Coomassie stained SDS gel and ubiquitin blot. B, Phospho ribosylation or ADP-ribosylation of ubiquitin completely abolishes *in vitro* ubiquitination by the HECT-type E3 ligase SopA. C, Phospho ribosylation or ADPribosylation of ubiquitin completely abolishes *in vitro* ubiquitination by the RING type E3 ligase Trim56.

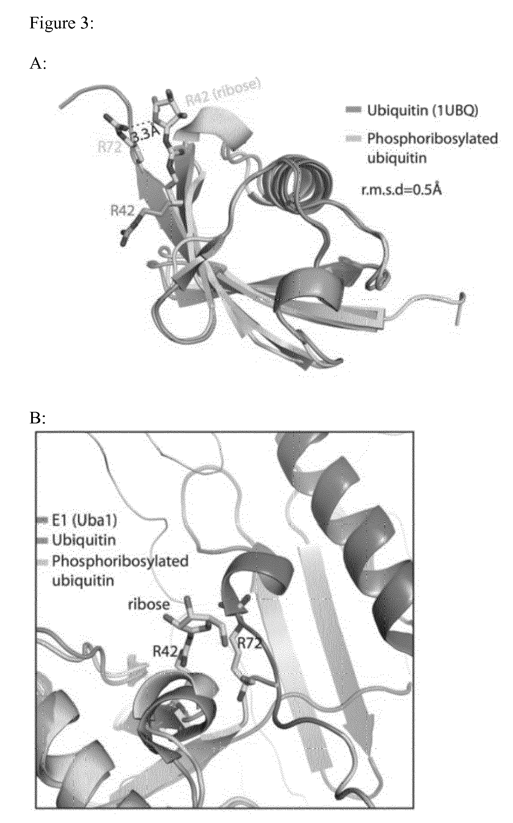

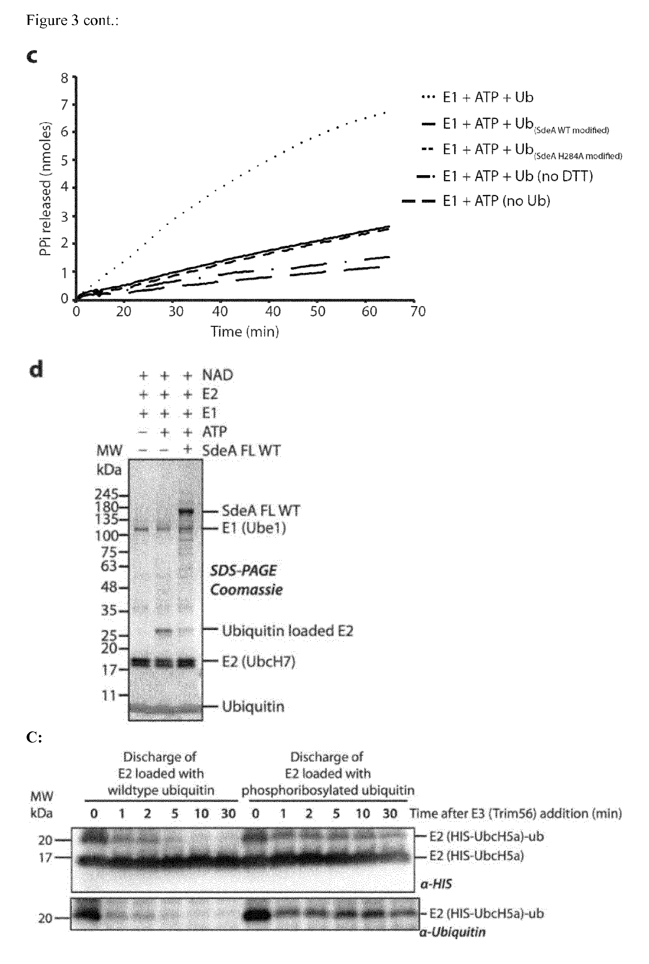

- **Figure 3:**: **Molecular basis for the inhibition of ubiquitin system by SdeA; A**, Crystal structure of phospho-ribosylated ubiquitin (shown in yellow) overlayed onto the wildtype ubiquitin structure (shown in green, PDB: 1UBQ). Phosphoribosylated arginine 42 and the interacting arginine 72 of the modified ubiquitin structure are shown. **B,** Superimposition of phosphoribosylated ubiquitin with wildtype ubiquitin in complex with yeast Uba1 (PDB:3CMM) showing the potential clashes between the phosphoribosyl moiety attached to Arginine 42 of ubiquitin and E1. **C,** Pyrophospate (PPi) release assay was performed with ubiquitin activating enzyme E1 and wildtype ubiquitin or modified ubiquitin. The plot shows nanomoles of PPi released over time. Both phosphoribosylated ubiquitin and ADP-ribosylated ubiquitin were defective in inducing the ATP hydrolysis by E1. **D,** E1, E2 and wildtype ubiquitin or phosphoribosylated ubiquitin were incubated with ATP. Transthiolesterification or ubiquitin transfer between the active sites of E1 and E2 was monitored by SDS-PAGE of reaction mixture components followed by Coomassie staining. Phosphoribosyl ubiquitin exhibits poor transthiolesterification. **E,** E2 discharge assay showing the defect in E3 induced discharge of phosphoribosyl ubiquitin conjugated E2.

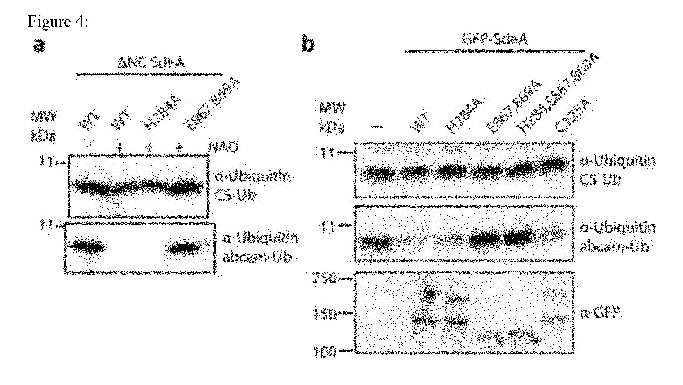

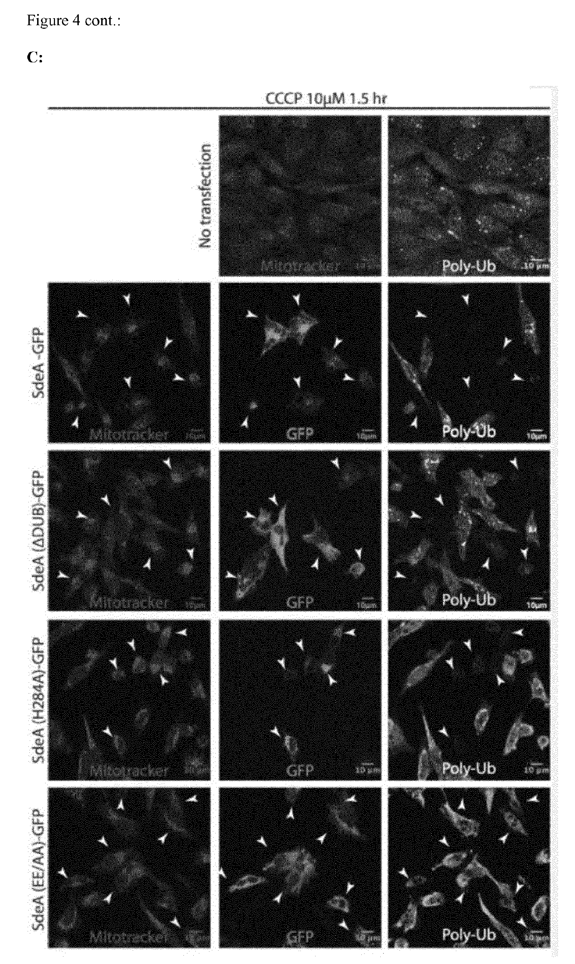

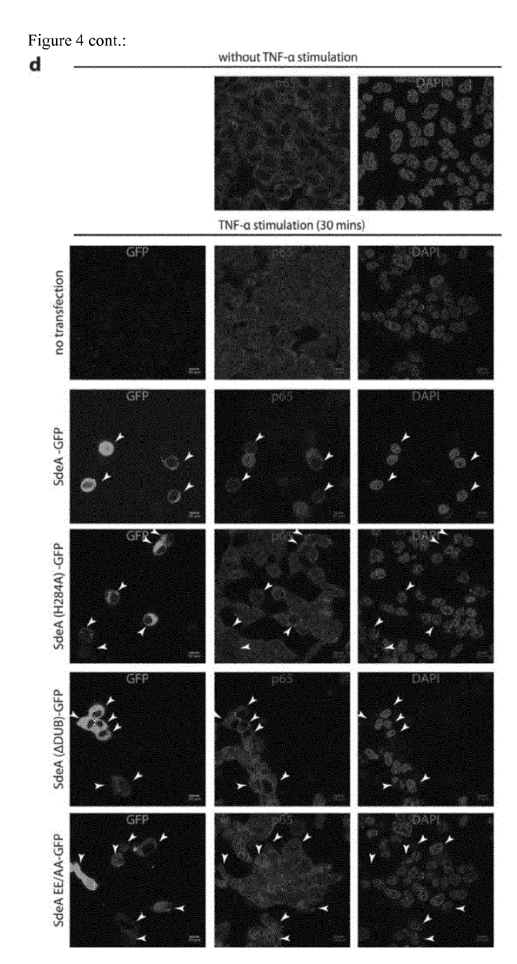

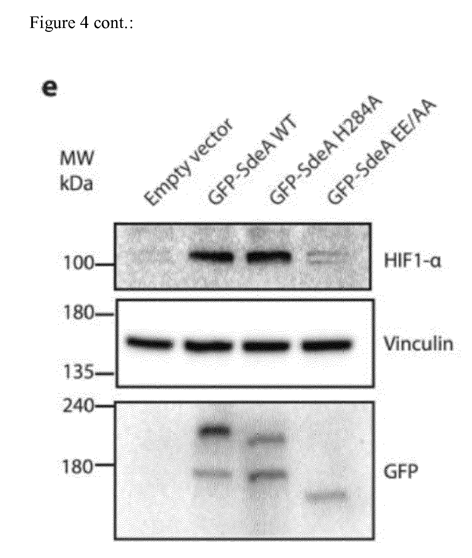

- **Figure 4:**: **SdeA phospho-ribosylates ubiquitin in cells and severely affects multiple ubiquitin-dependent processes. A**, wildtype ubiquitin and SdeA-modified ubiquitin are differentially recognized by two commercial ubiquitin antibodies. Antibody from cell signaling (CS-Ub) recognizes both modified and unmodified forms of ubiquitin equally well whereas the antibody from Abcam (abcamUb) selectively recognizes unmodified ubiquitin. Ubiquitin is modified by wildtype and various mutants of SdeA and probed with the above-mentioned antibodies. **B,** HeLa cells were transfected with wildtype and various mutants of GFP-SdeA. Cell lysates were subjected to SDS-PAGE and probed with CS-Ub and abcam-Ub antibodies to monitor ubiquitin modification. Analysis of ubiquitin signal from both antibodies depicts the dependence of ubiquitin modification on the mART motif of SdeA. *GFP tagged mART mutant shows greater electrophoretic mobility perhaps due to degradation of the FL protein. c, HeLa cells stably expressing HA-Parkin and ectopically expressing wildtype and various mutants of SdeA were treated with 10µM CCCP for 90 mins followed by staining with mitotracker and FK2 (Poly-Ub) antibody. SdeA WT, H284A, ΔNC expression leads to complete abolition of polyubiquitination at the mitochondria whereas expression of mART mutant (EE/AA) fails to inhibit polyubiquitination to the same level. GFP positive cells are marked with a white arrow. **d,** HeLa cells expressing wildtype and various mutants of SdeA were treated with TNF-α for 30 mins followed by staining with anti-p65 and DAPI. p65 translocation to nucleus is selectively blocked depending on SdeA's catalytic potency. GFP positive cells are marked with a white arrow. e, Under normoxic conditions, degradation of HIF1-α in HeLa cells expressing wildtype and various mutants of GFP-SdeA was probed. Expression of wildtype SdeA or H284A mutant leads to accumulation of HIF1-α.

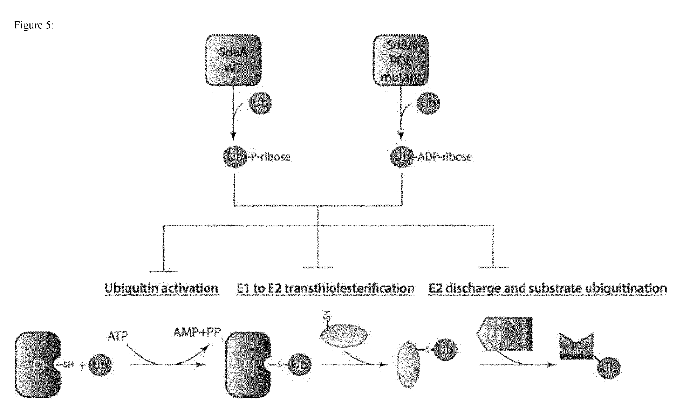

- **Figure 5:**: **Overall scheme for inhibition of ubiquitin system inhibition by SdeA.** SdeA wildtype (WT) protein phosphoribosylates ubiquitin. SdeA PDE mutant or ART domain alone ADP-ribosylates ubiquitin molecule (Figure 1). Both phosphoribosylation and ADP-ribosylation of ubiquitin lead to inhibition of multiple steps ubiquitination cascade as shown in figure 3. Eventually the modification of ubiquitin by SdeA leads to abrogation of ubiquitination *in vitro* and in cells (Figure 2 and Figure 4).

### And in the Sequence Listing:

SEQ ID NO: 1 SdeA full-length wildtype protein
SEQ ID NO: 2 mutated full-length EE/AA
SEQ ID NO: 3 mutated SdeA ΔNC
SEQ ID NO: 4 mutated SdeA ΔNC H284A
SEQ ID NO: 5 mutated SdeA ΔNC R344A
SEQ ID NO: 6 mutated SdeA ΔNC H288A
SEQ ID NO: 7 mutated SdeA ΔNC EE/AA

### EXAMPLES

### Materials and Methods

### Ubiquitin modification assays: (Figure 1)

For ubiquitin modification experiments, 25µM of purified untagged ubiquitin was incubated with 1-2µM of SdeA FL or ΔNC construct of SdeA in the presence or absence of 1mM NAD⁺ in a buffer containing 50mM Tris pH7.5, 50mM NaCl. The reaction mixture is subjected to SDS-PAGE or native PAGE followed by coomassie staining. Alternatively, reaction mixture was subjected to SDS-PAGE followed by western blotting using ubiquitin antibodies. CS-Ub is mouse monoclonal antibody against ubiquitin that was purchased from Cell Signaling (catalogue number: 3936s). Abcam-Ub is also a mouse monoclonal antibody against ubiquitin that was purchased from abcam (catalogue number: ab 7254). Biotin labeled NAD was purchased from Trevigen (catalogue number: 4670-500-01). Experiments using Biotin-NAD were analysed by transferring the reaction components onto a nitrocellulose membrane following the SDS-PAGE and probing the membrane with Streptavidin-HRP conjugate. 4µM of Rab33b was included in the reaction mixture where indicated. For producing the ADP-ribosylated ubiquitin, SdeA (H284A) was used instead of wildtype protein.

### In vitro ubiquitination reactions with E1, E2 and E3: (Fiugre 2)

*In vitro* ubiquitination reactions were performed as described previously (ref). Briefly, 0.2µM E1, 2µM E2 and 5µM E3 were incubated in the buffer 50mM Tris pH7.5, 50mM NaCl, 0.5mM DTT, 1mM MgCl2. Wildtype ubiquitin or SdeA-modified ubiquitin were used in the reaction mixtures. Reaction was initiated by the addition of 1mM ATP. For reactions using SopA as E3 ligase, its substrate Trim56 was added to the reactions. For reactions using Parkin, PINK1 was used to activate Parkin as previously described (Kazlauskaite et al., 2014). Both Parkin and PINK1 were purchased from MRC PPU reagents and services. The reactions were terminated by the addition of SDS-PAGE loading buffer. Ubiquitination was probed with SDS-PAGE analysis followed by commassie staining and/or western blotting followed by detection of polyubiquitination using a ubiquitin antibody.

### Purification, crystallization and structure determination of Phosphoribosylated ubiquitin: (Figure 3)

Phosphoribosylated ubiquitin was synthesized by an *in vitro* ADP ribosylation reaction consisting of 5µM SdeA wildtype (residues 200 to 1005), 165µM GST-tagged ubiquitin and NAD. The reaction was supplemented with 3C protease to cleave off the GST tag off the ubiquitin. The reaction was set up at 37 deg for 4 hours in a final volume of 2ml. The reaction mixture was subjected to size exclusion chromatography using a Superdex 75 (16/60) column that is pre-equilibriated with 10mM HEPES, 150mM NaCl and 1mM DTT. The fractions corresponding to ubiquitin were pooled and concentrated using a 3-kDa cut-off concentrator. The protein was then diluted with water to give a final buffer concentration of 1mM HEPES, 15mM NaCl and 0.1mM DTT and concentrated to 19 mg/ml for crystallization.

Crystals of phosphoribosylated ubiquitin were grown at 18 °C in 0.1M sodium acetate pH 4 - 5.5, 0.2M lithium sulfate and 30% PEG 8000. Crystals were flash cooled in liquid nitrogen using mother liquor supplemented with 15% glycerol as cryoprotectant. Diffraction data were collected at the Swiss light source (SLS, Villigen, Switzerland). The best crystal diffracted to a resolution of 1.8Å and belonged to the space group P12₁1, with 3 molecules in the asymmetric unit. Data were processed using the XDS package and the structure was solved by molecular replacement with ubiquitin (PDB:1ubq) in PHASER (Kabsch, 2010; Storoni, McCoy, & Read, 2004). The structure was refined with iterative rounds of manual rebuilding in COOT (Emsley & Cowtan, 2004) and maximum likelihood energy minimization and isotropic B-factor refinement in PHENIX (Adams et al., 2010). All structure figures were generated with PyMOL (The PyMOL Molecular Graphics System, Version 1.7.6.0 Schrodinger, LLC.).

### Pyrophosphate release assay and fluorescent ubiquitin-E1 loading assay: (Figure 3)

This assay was performed using EnzChek® Pyrophosphate Assay Kit from Molecular probes (catalogue number, E-6645) as per manufacturers instructions with slight modifications. Briefly, in final reaction volume of 300µL, 30 pmoles of E1, 10µM ubiquitin, 0.2mM 2-amino-6-mercapto-7-methylpurine ribonucleoside (MESG), 6µL of 3U/ml inorganic pyrophosphatase, 3µL of 100U/ml purine nucleoside phosphorylase were added in the buffer 50mM Tris pH7.5, 1mM MgCl2 and 50mM DTT where indicated. 1mM ATP was added to start the reaction and the enzymatic conversion of MESG to ribose 1-phosphate and 2-amino-6-mercapto-7-methylpurine was probed using the continuous measurement of the reaction mixture's absorbance at 360nm wavelength using a TECAN infinite® 200 Pro plate reader. The absorbance was converted into moles of PPi released based on the standard curve obtained using various amounts of sodium pyrophosphate.

### E2 loading and discharge assays: (Figure 3)

2µM of purified UbcH7 and 25µM ubiquitin was added to 0.2µM Ube1 in the reaction buffer containing 50mM Tris pH7.5 50mM NaCl and 1mM MgCl₂. E2-loading assays were started by the addition of 1mM ATP and the reaction was stopped after 10 mins by the addition of SDS-PAGE loading buffer and heating the samples at 96C for 2mins. Reaction mixture is then subjected SDS-PAGE followed by coomassie staining to assay the ubiquitin loading of E2. For E2 discharge assays, wildtype ubiquitin or phosphoribosylated ubiquitin loaded E2 (UbcH5a) was prepared as described above except that the reaction mixtures were incubated for 1 hour to allow complete loading of E2 with the modified ubiquitin. The reaction mixture was incubated with 1 unit of Apyrase for 30 minutes to completely deplete the ATP. 3µM E3 was then added to initiate the discharge of ubiquitin from E2. Samples were collected at indicated time points and SDS-PAGE loading buffer was added to stop the reaction. Reaction mixtures were subjected to SDS-PAGE, blotted and probed with antibodies against HIS tag and ubiquitin to monitor the discharge of ubiquitin loaded HIS-UbcH5a.

### Mitophagy, TNF-α experiments and probing HIF-1α levels: (Figure 4)

4×10⁵ HeLa Flp-In TRex HA Parkin cells were seeded on a sterile coverslip placed in a 6 well plate. After allowing the cells to settle overnight, GFP-SdeA plasmids were transfected using Polyethyleneimine. 24 hours after transfection, cells were checked for the expression of SdeA with green fluorescence. 1 µg/mL of Doxcyclin (Clontech) n was added to the cells to induce the expression of HA-Parkin. After 6 hours of treatment with Doxycyclin, mitochondria were stained with 100nM MitoTracker (Invitrogen). Cells were then treated with 10µM CCCP for 90 minutes to induce mitophagy. Slides were then fixed with formaldehyde and stained with FK2 antibody (EMD-Millipore) using standard procedures. Cells surrounding the glas coverslip were lysed and probed for HA-Parkin and GFP-SdeA using HA and GFP antibodies respectively. For TNF-α experiments, HeLa cells were transfected with various SdeA constructs as described above. 24 hours after the transfection, the culture medium was changed to DMEM minus FBS to starve cells overnight. 25 ng/mL of TNF-α was added to induce the signaling. After 20 minutes, cells were fixed and stained for p65 and DAPI using standard procedures. For the HIF-1α experiment, HeLa cells were cultures and transfected with various SdeA constructs as described above. 24 hours after the transfection, cells were lysed and the levels of HIF-1α were probed with antibody against the protein (R&D systems).

### Results

It was observed that in *in vitro* assays with both full-length (FL) SdeA and ΔNC construct of SdeA, the entire ubiquitin pool that is present in ~20-fold molar excess when compared to Rab33b is modified in NAD⁺-dependent manner as seen by native-PAGE of the reaction components (Fig. 1a). This showed that the modification of ubiquitin is not merely an intermediate step in the ubiquitination of Rab33b but SdeA is actively modifying ubiquitin while also using a subset of the modified pool to ubiquitinate Rab33b.

Previous structural and biochemical studies have shown that SdeA contains an N-terminal deubiquitinase (DUB) domain spanning residues 1 to 200 and a mono ADP-ribosyl transferase (mART) domain spanning residues 500 to 1000 (Qiu et al., 2016; Sheedlo et al., 2015). A domain prediction analysis of SdeA revealed that the region between residues 200 and 500 is similar to the phosphodiesterase (PDE) domain in the LP effector 1pg1496 (Fig. 1b). Using biotin labeled NAD (biotin is attached to the amino group of adenine base) in ubiquitin modification reactions, the inventors monitored the phosphodiester bond cleavage of ADP-ribosylated ubiquitin (Fig. 1c). SdeA construct spanning residues 200 to 1005 (ΔNC SdeA) was used in these ubiquitin modification reactions (Fig. 1b). As probed by streptavidin blot, it was found that the PDE domain mutant proteins H284A, H288A and R344A are defective in the cleavage of phosphodiester bond in ADP-ribosylated ubiquitin leading to accumulation of ADP-ribosylated ubiquitin. Whereas wildtype protein completely processed ADP-ribosylated ubiquitin resulting in the formation of phosphoribosylated ubiquitin (Fig. 1d). As expected, the mART domain mutant (E867A, E869A or EE/AA) of SdeA failed to modify ubiquitin.

Next it was sought to identify the effect of SdeA WT or PDE domain mutant mediated phosphoribosylation or ADP-ribosylation of ubiquitin in regulating the ubiquitin system. Towards this, an *in vitro* ubiquitin ligase activity assay was performed with RING-between-RING (RBR) type E3 ligase Parkin. Use of both phosphoribosylated and ADP-ribosylated ubiquitin resulted in complete inhibition of Parkin's activity (Fig. 2a). Similar results were obtained with two unrelated ubiquitin ligases, one belonging to the RING type (TRIM56) and another belonging to the HECT type (*Salmonella* effector, SopA) E3 ligase class (Fig. 2b, 2c). The lack of activity of three different classes of E3 ligases with SdeA-modified ubiquitin points towards defective E1 or E2 related steps of the ubiquitin reaction cascade. To understand the molecular basis of the ubiquitin system shutdown by SdeA, the crystal structure of phosphoribosylated ubiquitin was determined at 1.8Å resolution (Fig. 3a). Comparison of wildtype ubiquitin structure (pdb:1ubq) with the structure of the phosphoribosylated form of ubiquitin revealed that Arg42 and Arg72 containing beta strands are shifted from their default position (Fig. 3a). This is a result of the side chain of Arg72 making polar contacts with the ribosyl moiety attached to Arg42. Interestingly, both Arg42 and Arg72 of ubiquitin have been previously implicated in ubiquitin activation by E1. Furthermore, Arg42 and Arg72 are directly involved in ionic interactions with E1 as seen in the yeast Uba1-ubiquitin complex structure (Lee & Schindelin, 2008). Aligning the phosphoribosylated ubiquitin structure with ubiquitin bound to Uba1 demonstrated major steric clashes between modified ubiquitin and E1 (Fig. 3b). Using an enzyme-coupled spectrophotometric assay, the inventors measured the kinetics of pyrophosphate (PPi) release during ubiquitin activation reaction using E1 and ubiquitin/ SdeA-modified ubiquitin. In agreement with the structural analysis of SdeA-modified ubiquitin, both phosphoribosylated ubiquitin and ADP-ribosylated ubiquitin exhibited markedly reduced PPi release compared to wildtype ubiquitin in ubiquitin-activation reactions (Fig. 3c). Furthermore, SdeA-modified ubiquitin is also defective in transfer of ubiquitin from E1 to E2 via transthiolesterification as seen from the coomassie stained SDS-PAGE analysis of E2 loaded ubiquitin (Fig. 3d). Also the E3-mediated ubiquitin discharge from E2 is compromised with phosphoribosylated ubiquitin (Fig. 3e), indicating multiple steps of the cellular ubiquitination cascade are defective with modified ubiquitin.

The inventors then checked if ectopically expressed SdeA could modify the cellular ubiquitin pool and affect ubiquitination in cells. Towards this, a strategy was developed to assay phosphoribosylation of ubiquitin in cells. While screening different ubiquitin antibodies, the inventors noticed that the ubiquitin antibody from Cell Signaling Technology^{®} (referred as CS-Ub antibody) recognizes both modified and unmodified ubiquitin with the same efficiency whereas the antibody from abcam^{®} (referred as abcam-Ub antibody) selectively recognizes only the unmodified ubiquitin and fails to recognize the modified ubiquitin (Fig. 4a). The inventors expressed GFP tagged SdeA wildtype, H284A and the mART motif mutant EE/AA in Hela cells and probed for any modification of ubiquitin using the above mentioned pair of antibodies. Expression of SdeA wildtype or H284A mutant but not the mART mutant led to the modification of ubiquitin in cells (Fig. 4b).

Next, the inventors sought to determine if the modification of ubiquitin compromises ubiquitin-dependent processes in cells. Ubiquitin is central to many cellular processes including protein homeostasis, autophagy and signaling (Hershko, Ciechanover, & Varshavsky, 2000). The inventors first checked the effect of SdeA expression on mitophagy, a selective autophagy process for the clearance of damaged mitochondria. Ubiquitination of mitochondrial membrane protein fraction by the E3 ubiquitin ligase Parkin is one of the crucial events leading to the autophagy of mitochondria (Narendra, Tanaka, Suen, & Youle, 2008). They treated Hela cells stably expressing HA-Parkin with carbonyl cyanide *m*-chlorophenyl hydrazine (CCCP) to induce mitophagy. Using immunofluorescence microscopy, they then looked for polyubiquitination at mitochondria using FK2 anti-ubiquitinated proteins antibody and mitotracker. In cells with no SdeA transfection, CCCP treatment effectively induced polyubiquitination at the mitochondria (Fig. 4c). Expression of WT SdeA or H284A mutant severely impaired polyubiquitination at the mitochondria. Levels of HA-Parkin are similar in all the experiments indicating that the lack of polyubiquitination is a direct consequence of SdeA overexpression in cells (Extended data Fig. 4b). Surprisingly, the general background signal for polyubiquitin chains in the cytoplasm also diminished greatly in SdeA expressing cells indicating a general defect in the cellular ubiquitin system. Similar results were obtained with the expression of ΔNC construct of SdeA lacking the N-terminal DUB domain indicating that the SdeA's inhibitory effect on the ubiquitin system is mediated mainly through its mART domain. Accordingly, expression of the mART mutant did not lead to a strong decrease in polyubiquitination at the mitochondria. To further examine the functional consequences of ubiquitin modification by SdeA the inventors turned to tumor necrosis factor-α (TNF-α) signaling. TNF signaling plays crucial roles in innate immune response, cell death and cancer (Wajant, Pfizenmaier, & Scheurich, 2003). Ubiquitination of multiple substrates at various stages of TNF signaling is critical to signal transduction and eventual nuclear translocation of the transcription factor p65 leading to induction of its target gene expression (Walczak, 2011). They tested if SdeA expression in cells can affect TNF signaling (Fig 4d). As expected, p65 translocated to nucleus upon TNF-α induction in control cells. Expression of wildtype SdeA or H284A mutant or ΔNC construct completely abolished p65 nuclear translocation, whereas expression of the mART mutant did not noticeably affect the translocation of p65 to nucleus. The inventors then tested the effect of SdeA expression on proteasomal degradation of intracellular proteins. Hypoxia inducible factor 1-α (HIF1-α) is a universal regulator of physiological responses to hypoxia and is overexpressed in many cancers (Weidemann & Johnson, 2008). Under normoxic conditions, HIF1-α constantly undergoes von Hippel-Lindau protein (pVHL) mediated polyubiquitination and subsequently gets degraded by the ubiquitin-proteasome system. Expression of SdeA WT or H284A mutant but not the mART mutant led to HIF1-α stabilization in Hela cells (Fig. 4e). Our results demonstrate that the SdeA-mediated phosphoribosylation of ubiquitin is a potent inhibitor of the cellular ubiquitin system and severely affects multiple ubiquitin-dependent pathways.

In summary, SdeA wildtype phosphosribosylates ubiquitin and SdeA PDE mutants ADP-ribosylate ubiquitin. the inventors prove that both phosphoribosylation and ADP-ribosylation of ubiquitin make it unsuitable for multiple steps in the ubiquitination reaction cascade and abrogate ubiquitination both *in vitro* and in cells (Figure 5).

### References:

Adams, P. D., Afonine, P. V., Bunkóczi, G., Chen, V. B., Davis, I. W., Echols, N., et al. (2010). PHENIX: a comprehensive Python-based system for macromolecular structure solution. Acta Crystallographica Section D: Biological Crystallography, 66(Pt 2), 213-221. http://doi.org/10.1107/S0907444909052925 E
msley, P., & Cowtan, K. (2004). Coot: model-building tools for molecular graphics. Acta Crystallographica Section D: Biological Crystallography, 60(Pt 12 Pt 1), 2126-2132. http://doi.org/10.1107/S0907444904019158
Hershko, A., Ciechanover, A., & Varshavsky, A. (2000). The ubiquitin system. Nature Medicine, 6(10), 1073-1081. http://doi.org/10.1038/80384
Kabsch, W. (2010). XDS. Acta Crystallographica Section D: Biological Crystallography, 66(Pt 2), 125-132. http://doi.org/10.1107/50907444909047337 Ka
zlauskaite, A., Kondapalli, C., Gourlay, R., Campbell, D. G., Ritorto, M. S., Hofmann, K., et al. (2014). Parkin is activated by PINK1-dependent phosphorylation of ubiquitin at Ser65. Biochemical Journal, 460(1), 127-141. http://doi.org/10.1042/BJ20140334
Lee, I., & Schindelin, H. (2008). Structural Insights into E1-Catalyzed Ubiquitin Activation and Transfer to Conjugating Enzymes. Cell, 134(2), 268-278. http://doi.org/10.1016/j.ce11.2008.05.046
Narendra, D., Tanaka, A., Suen, D.-F., & Youle, R. J. (2008). Parkin is recruited selectively to impaired mitochondria and promotes their autophagy. The Journal of Cell Biology, 183(5), 795-803. http://doi.org/10.1083/jcb.200809125
Qiu, J., Sheedlo, M. J., Yu, K., Tan, Y., Nakayasu, E. S., Das, C., et al. (2016). Ubiquitination independent of E1 and E2 enzymes by bacterial effectors. Nature, 533(7601), 120-124. http://doi.org/10.1038/nature17657
Sheedlo, M. J., Qiu, J., Tan, Y., Paul, L. N., Luo, Z.-Q., & Das, C. (2015). Structural basis of substrate recognition by a bacterial deubiquitinase important for dynamics of phagosome ubiquitination. Proceedings of the National Academy of Sciences of the United States of America, 112(49), 15090-15095. http://doi.org/10.1073/pnas.1514568112 St
oroni, L. C., McCoy, A. J., & Read, R. J. (2004). Likelihood-enhanced fast rotation functions. Acta Crystallographica Section D: Biological Crystallography, 60(Pt 3), 432-438. http://doi.org/10.1107/S0907444903028956
Wajant, H., Pfizenmaier, K., & Scheurich, P. (2003). Tumor necrosis factor signaling. Cell Death & Differentiation, 10(1), 45-65. http://doi.org/10.1038/sj.cdd.4401189 W
alczak, H. (2011). TNF and ubiquitin at the crossroads of gene activation, cell death, inflammation, and cancer. Immunological Reviews, 244(1), 9-28. http://doi.org/10.1111/j.1600-065X.2011.01066.x
Weidemann, A., & Johnson, R. S. (2008). Biology of HIF-11|[alpha]|. Cell Death & Differentiation, 15(4), 621-627. http://doi.org/10.1038/cdd.2008.12

## Claims

1. An *in vitro* method of deactivating, blocking and/or impairing the ubiquitin system in a cell, comprising introducing into the cell a recombinant SdeA protein, or a recombinant SdeA protein derivative, wherein the SdeA protein, or the recombinant SdeA protein derivative, comprises at least an ADP-ribosyl transferase domain.

2. The method according to claim 1, wherein said recombinant SdeA protein, or a recombinant SdeA protein derivative, comprises a phosphodiesterase wherein residue corresponding to H284 and/or R344 in SEQ ID NO: 1 is mutated, preferably is H284A, and/or R344A, respectively.

3. A mutated SdeA protein, comprising at least a functional ADP-ribosyl transferase domain, and not comprising a functional phosphodiesterase domain.

4. The mutated SdeA protein according to claim 3, comprising an amino acid sequence having at least 80% sequence identity to any of the sequences shown in SEQ ID NO: 1 to 7.

5. The mutated SdeA protein according to claim 3 or 4, comprising a non-functional phosphodiesterase domain, wherein the residue corresponding to H284 in SEQ ID NO: 1 is mutated, preferably is H284A.

6. A nucleic acid construct, comprising a nucleic acid sequence encoding the mutated SdeA protein according to any of claims 3 to 5.

7. A recombinant cell comprising a mutated SdeA protein according to any one of claims 3 to 5, or a nucleic acid construct according to claim 6.

8. An *in vitro* method of deactivating, blocking or impairing the ubiquitin system in a cell, comprising contacting the cell with one of (i) a phospho-ribosylated ubiquitin, or (ii) an ADP-ribosylated ubiquitin.

9. An *in vitro* method of modifying ubiquitin, or ubiquitin-like protein, comprising the step of contacting the ubiquitin, or ubiquitin-like protein, with a an SdeA protein, or a mutated SdeA protein according to any of claims 3 to 5, preferably in the presence of NAD.

10. A compound for use in medicine, wherein the compound is a mutated SdeA protein according to any of claims 3 to 5, a nucleic acid construct according to claim 6, a cell according to claim 7, or a phospho-ribosylated or ADP-ribosylated ubiquitin.

11. The compound for use according to claim 10, for use in the treatment of a tumor disease, or a disease associated with a pathological ubiquitination, proteasomal degradation, mitophagy, autophagy or TNF mediated cell signaling.

12. An *in vitro* method for identifying a modulator of ubiquitination, comprising a step of contacting a ubiquitin protein, or ubiquitin-like protein, with a candidate SdeA variant protein in the presence of NAD and subsequently determining the level of ADP-ribosylation and/or phospho-ribosylation of said ubiquitin protein, or ubiquitin-like protein.

13. A phosphodiesterase inhibitor for use in the treatment of an infectious disease.

14. The phosphodiesterase inhibitor for use according to claim 13, which is a compound that is capable of inhibiting or reducing selectively or non-selectively the activity of a phosphodiesterase.

15. A method for screening for anti-infectious compounds, the method comprising the steps of bringing a candidate compound into contact with a SdeA protein and a diphosphate substrate under conditions that allow for an enzymatically catalyzed hydrolysis of the diphosphate substrate, and comparing the level of hydrolysis of the diphosphate substrate in the presence of the candidate compound with a control, wherein a reduced level of hydrolysis indicates that the compound is an anti-infectious compound.
